# EUROPEAN PATENT APPLICATION

(11) **EP 4 763 054 A1**
(43) Date of publication of application: **24.06.2026**
(21) Application number: 24911574.2
(22) Date of filing: 28.12.2024
(51) Int. Cl.: A61B 5/00, A61B 5/145

(54) **BODY INDEX MONITORING METHOD, USER INTERFACE AND RELATED APPARATUS**

(30) Priority: 29.12.2023 CN 202311872486
(71) Applicant: HUAWEI TECHNOLOGIES CO., LTD., Shenzhen 518129 (CN)
(72) Inventor: LIU, Xiangyu, Shenzhen, Guangdong 518129 (CN)
(74) Representative: Maiwald GmbH
(86) International application number: PCT/CN2024/143472
(87) International publication number: WO 2025/140660

(57) **Abstract**

This application discloses a body indicator monitoring method, a user interface, and a related apparatus. In the method, a first device may receive a first operation, and send first information to a third device in response to the first operation. After obtaining the first information, the third device may send an obtained value of a first body indicator to a second device, and then the second device outputs the value of the first body indicator, or outputs alarm information when the value of the first body indicator exceeds a first range. It can be learned that, based on the method, by performing an operation on a device other than the third device, the value that is of the body indicator and that is obtained by the third device can be obtained, or the alarm information can be obtained when the body indicator is abnormal, to facilitate an operation of a user, thereby meeting a body indicator monitoring requirement of the user.

## Description

This application claims priority to Chinese Patent Application No. 202311872486.3, filed with the China National Intellectual Property Administration on December 29, 2023 and entitled "BODY INDICATOR MONITORING METHOD, USER INTERFACE, AND RELATED APPARATUS", which is incorporated herein by reference in its entirety.

### TECHNICAL FIELD

This application relates to the field of terminals, and in particular, to a body indicator monitoring method, a user interface, and a related apparatus.

### BACKGROUND

With continuous progress of science and technology, a growing number of devices are designed to be worn on users, and are configured to measure body indicators of the users, for example, blood glucose, a heart rate, a body temperature, blood pressure, blood lipid, and blood ketone. Common devices include a continuous glucose monitoring (continuous glucose monitoring, CGM) device, where the device may be configured to measure blood glucose of a user, a continuous ketone monitoring (continuous ketone monitoring, CKM) device, where the device may be configured to measure blood ketone of the user, a continuous lactate monitoring (continuous lactate monitoring, CLM) device, where the device may be configured to measure lactate of the user, and an electrocardiogram patch, where the electrocardiogram patch is configured to measure an electrocardiogram signal of the user.

Currently, due to constraints on space, power consumption, and wearing positions, various types of devices configured to measure body indicators have limited interaction manners. Values that are of the body indicators and that are obtained by the devices are mainly synchronized to a receiving device such as a dedicated reader/writer, a mobile phone, or a watch for displaying. However, a user needs to hold the receiving device close to the devices that measure the body indicators, to obtain these values by using a near field communication (near field communication, NFC) technology. Therefore, a user operation is troublesome, and cannot meet a requirement of the user for conveniently monitoring the body indicators.

### SUMMARY

This application provides a body indicator monitoring method, a user interface, and a related apparatus. Based on the method, a related interaction manner in which a user monitors a body indicator by using an electronic device can be extended, and a requirement of the user for conveniently monitoring the body indicator is met.

According to a first aspect, an embodiment of this application provides a body indicator monitoring method. The method includes: A first device receives a first operation; the first device sends first information to a third device in response to the first operation; the third device sends an obtained value of a first body indicator to a second device after receiving the first information; and the second device outputs the value of the first body indicator, or outputs alarm information when the value of the first body indicator exceeds a first range.

For example, the first device and the second device may be a same device, or may be different devices. The first device and the third device may be devices used by a same user, or may be devices used by different users. If the first device and the second device are different devices, the first device and the second device may be devices used by a same user, or may be devices used by different users.

Based on the method provided in the first aspect, the user does not need to directly contact or approach a device configured to monitor a body indicator of a patient, that is, the third device, but can trigger, by using another device, body indicator monitoring, to facilitate an operation of the user. In addition, a body status of the patient may be monitored by outputting the value of the body indicator of the patient, or outputting the alarm information when the body indicator of the patient is abnormal, to meet a requirement of the user for monitoring a plurality of body indicators, thereby improving user experience of monitoring body indicators.

With reference to the first aspect, in an implementation, when the first operation is a first body movement, the second device outputs the value of the first body indicator; or, when the first operation is a second body movement, the second device outputs the alarm information when the value of the first body indicator exceeds the first range.

It can be learned that the user may trigger, by initiating different body movements, the second device to output different body indicator monitoring modes, to meet different body indicator monitoring requirements of the user.

With reference to the first aspect, in an implementation, the first device and the third device are devices used by a same user, the first operation is a body movement, and before the third device sends the obtained value of the first body indicator to the second device, the method further includes: The third device receives the first operation.

In this way, the operation of the user can be identified collaboratively by using a plurality of devices, to avoid misidentification, thereby implementing accurate body indicator broadcasting.

With reference to the first aspect, in an implementation, before the second device outputs the alarm information when the value of the first body indicator exceeds the first range, the method further includes: The first device or the second device determines that a value of a second body indicator of the user wearing the third device exceeds a second range, where the second body indicator is related to the first body indicator.

In this way, the body status of the patient may be evaluated with reference to a plurality of factors, to avoid a false alarm of the second device, reduce a quantity of alarms of the second device, and improve accuracy of monitoring the body indicator of the patient.

With reference to the first aspect, in an implementation, the value of the first body indicator is the value that is of the body indicator and that is obtained by the third device in real time, the second device outputs the value of the first body indicator, and the second device is a device that establishes a communication connection to the first device and a device with best privacy in the first device.

In other words, if the second device outputs the value of the first body indicator, a device with best privacy may be selected as the second device, to ensure, to the greatest extent, privacy when the body indicator of the patient is broadcast.

With reference to the first aspect, in an implementation, the first operation includes a first posture of the user; and that the first device receives the first operation specifically includes: The first device obtains the first posture by using a camera.

In other words, the user may trigger, by making a specified posture, the electronic device to monitor the body indicator, to extend a related interaction manner in which the user monitors the body indicator by using the electronic device.

With reference to the first aspect, in an implementation, that the second device outputs the value of the first body indicator specifically includes: The second device displays the value of the first body indicator, and/or displays a first body indicator curve, where the first body indicator curve includes a plurality of values of the first body indicator that are obtained by the third device.

In this way, the electronic device can display the body indicator status of the user from different aspects.

According to a second aspect, an embodiment of this application provides a body indicator monitoring method, where a first device and a third device are devices used by a same user, and the method includes: The first device sends first information to the third device when a value that is of a second body indicator and that is obtained by the first device exceeds a second range or the first device receives a first movement; the third device sends an obtained value of a first body indicator to a second device after receiving the first information, where the first body indicator is related to the second body indicator; and the second device outputs alarm information when the value of the first body indicator exceeds a first range.

Based on the method provided in the second aspect, when another body indicator of the user is abnormal or when the user performs some movements, the electronic device may trigger body indicator monitoring on the user, and remind the user to pay attention to an abnormal body status of the user in a timely manner, so that the user can take measures in a timely manner to ensure body health of the user.

With reference to the first aspect and the second aspect, in an implementation, the second device outputs the alarm information when the value of the first body indicator exceeds the first range, and the second device is a device that is being used by the user, and the second device is a device that establishes a communication connection to the first device and/or the first device.

In other words, if the user triggers the second device to output the alarm information when the body indicator of the patient is abnormal, the device may be determined based on timeliness of receiving the information by the user, to prevent the user from missing the alarm information.

With reference to the first aspect and the second aspect, in an implementation, the first device includes a first electrode, the third device includes a second electrode, and both the first electrode and the second electrode are in contact with skin of the user; and that the first device sends the first information to the third device specifically includes: The first device sends the first information to the second electrode of the third device by using the first electrode.

It can be learned that in this embodiment of this application, human skin communication is combined with body indicator monitoring, and a body indicator monitoring request may be transmitted between the electronic devices in a manner of human skin communication, to reduce a data leakage risk.

With reference to the first aspect and the second aspect, in an implementation, the second device includes a third electrode, the third device includes a fourth electrode, and both the third electrode and the fourth electrode are in contact with the skin of the user; and that the third device sends the obtained value of the first body indicator to the second device specifically includes: The third device sends, to the third electrode of the second device by using the fourth electrode, the value that is of the first body indicator and that is obtained by the third device.

It can be learned that in this embodiment of this application, human skin communication is combined with body indicator monitoring, and the electronic device may transmit a body indicator status to the user in a manner of human skin communication, to reduce a risk of data leakage of the body indicator, thereby protecting user privacy.

In an implementation, the first electronic device and the third electrode may be a same electrode, and the second electrode and the fourth electrode may be a same electrode.

With reference to the first aspect and the second aspect, in an implementation, the first device and the second device are wearable devices, smart home devices, mobile phones, tablets, or computers.

With reference to the first aspect and the second aspect, in an implementation, the value of the first body indicator includes one or more values, and the value indicates a current actual status of the first body indicator of the user, or indicates a current-historical change status of the first body indicator of the user.

With reference to the first aspect and the second aspect, in an implementation, the third device is a continuous glucose monitoring CGM device, and the first body indicator is blood glucose; or the third device is a continuous ketone monitoring CKM device, and the first body indicator is blood ketone; or the third device is a continuous lactate monitoring CLM device, and the first body indicator is lactate; or the third device is an electrocardiogram patch, and the first body indicator is an electrocardiogram signal.

According to a third aspect, an embodiment of this application provides a body indicator monitoring method. The method includes: A first device receives a first operation; the first device sends first information to a third device in response to the first operation; the first device receives a value that is of a first body indicator and that is obtained by the third device; and the first device outputs the value of the first body indicator, or outputs alarm information when the value of the first body indicator exceeds a first range.

Based on the method provided in the third aspect, the user does not need to directly contact or approach a device configured to monitor a body indicator of a patient, that is, the third device, but can trigger, by using another device, body indicator monitoring, and view a body indicator status of the user by using the device, to facilitate an operation of the user. In addition, a body status of the patient may be monitored by outputting the value of the body indicator of the patient, or outputting the alarm information when the body indicator of the patient is abnormal, to meet a requirement of the user for monitoring a plurality of body indicators, thereby improving user experience of monitoring body indicators.

With reference to the third aspect, in an implementation, when the first operation is a first body movement, the first device outputs the value of the first body indicator; or, when the first operation is a second body movement, the first device outputs the alarm information when the value of the first body indicator exceeds the first range.

With reference to the third aspect, in an implementation, before the first device outputs the alarm information when the value of the first body indicator exceeds the first range, the method further includes: The first device determines that a value of a second body indicator of a user wearing the third device exceeds a second range, where the second body indicator is related to the second body indicator.

With reference to the third aspect, in an implementation, the first operation includes a first posture of the user; and that the first device receives the first operation specifically includes: The first device obtains the first posture by using a camera.

With reference to the third aspect, in an implementation, that the first device outputs the value of the first body indicator specifically includes: The first device displays the value of the first body indicator, and/or displays a first body indicator curve, where the first body indicator curve includes a plurality of values of the first body indicator that are obtained by the third device.

According to a fourth aspect, an embodiment of this application provides a body indicator monitoring method, where a first device and a third device are devices used by a same user, and the method includes: The first device sends first information to the third device when a value that is of a second body indicator and that is obtained by the first device exceeds a second range or the first device receives a first movement, where the first information indicates the third device to send a value that is of a first body indicator and that is obtained by the third device; the first device receives the value of the first body indicator, where the first body indicator is related to the second body indicator; and the first device outputs alarm information when the value of the first body indicator exceeds a first range.

Based on the method provided in the fourth aspect, when another body indicator of the user is abnormal or when the user performs some movements, the electronic device may trigger body indicator monitoring on the user, and remind the user to pay attention to an abnormal body status of the user in a timely manner, so that the user can take measures in a timely manner to ensure body health of the user.

With reference to the third aspect and the fourth aspect, in an implementation, the first device includes a first electrode, and the first electrode is in contact with skin of the user; and that the first device sends the first information to the third device specifically includes: The first device sends the first information to the third device by using the first electrode.

With reference to the third aspect and the fourth aspect, in an implementation, the first device includes a third electrode, and the third electrode is in contact with the skin of the user; and that the first device receives the value that is of the first body indicator and that is obtained by the third device specifically includes: The first device receives, by using the third electrode, the value that is of the first body indicator and that is obtained by the third device.

In an implementation, the first electrode and the third electrode may be a same electrode.

With reference to the third aspect and the fourth aspect, in an implementation, the value of the first body indicator includes one or more values, and the value indicates a current actual status of the first body indicator of the user, or indicates a current-historical change status of the first body indicator of the user.

With reference to the third aspect and the fourth aspect, in an implementation, the third device is a continuous glucose monitoring CGM device, and the first body indicator is blood glucose; or the third device is a continuous ketone monitoring CKM device, and the first body indicator is blood ketone; or the third device is a continuous lactate monitoring CLM device, and the first body indicator is lactate; or the third device is an electrocardiogram patch, and the first body indicator is an electrocardiogram signal.

According to a fifth aspect, an embodiment of this application provides a communication system, where the communication system includes a first device, a second device, and a third device, where the first device is configured to: receive a first operation, and send first information to the third device in response to the first operation; the third device is configured to send an obtained value of a first body indicator to the second device after receiving the first information; and the second device is configured to: output the value of the first body indicator, or output alarm information when the value of the first body indicator exceeds a first range.

According to a sixth aspect, an embodiment of this application provides a communication system, where the communication system includes a first device, a second device, and a third device, and the first device and the third device are devices used by a same user, where the first device is configured to send first information to the third device when a value that is of a second body indicator and that is obtained by the first device exceeds a second range or the first device receives a first movement; the third device is configured to send an obtained value of a first body indicator to the second device after receiving the first information, where the first body indicator is related to the second body indicator; and the second device is configured to output alarm information when the value of the first body indicator exceeds a first range.

According to a seventh aspect, an embodiment of this application provides an electronic device, including a memory, one or more processors, and one or more programs, where when the one or more processors execute the one or more programs, the electronic device is enabled to implement the method implemented by the first device, the second device, or the third device in any one of the first aspect or the implementations of the first aspect, or the electronic device is enabled to implement the aspect implemented by the first device, the second device, or the third device in any one of the second aspect or the implementations of the second aspect, or the electronic device is enabled to implement the method described in any one of the third aspect or the implementations of the third aspect, or in any one of the fourth aspect or the implementations of the fourth aspect.

According to an eighth aspect, an embodiment of this application provides a computer-readable storage medium, including instructions. When the instructions are run on an electronic device, the electronic device is enabled to implement the method implemented by the first device, the second device, or the third device in any one of the first aspect or the implementations of the first aspect, or the electronic device is enabled to implement the aspect implemented by the first device, the second device, or the third device in any one of the second aspect or the implementations of the second aspect, or the electronic device is enabled to perform the method described in any one of the third aspect or the implementations of the third aspect, or in any one of the fourth aspect or the implementations of the fourth aspect.

According to a ninth aspect, an embodiment of this application provides a computer program product. When the computer program product is run on a computer, the computer is enabled to implement the method implemented by the first device, the second device, or the third device in any one of the first aspect or the implementations of the first aspect, or the computer is enabled to implement the aspect implemented by the first device, the second device, or the third device in any one of the second aspect or the implementations of the second aspect, or the computer is enabled to perform the method described in any one of the third aspect or the implementations of the third aspect, or in any one of the fourth aspect or the implementations of the fourth aspect.

For descriptions of beneficial effects in the second aspect to the ninth aspect, refer to descriptions of the beneficial effects in the first aspect.

### BRIEF DESCRIPTION OF DRAWINGS

FIG. 1 is a diagram of a communication system 1000 according to an embodiment of this application;
FIG. 2 is a schematic flowchart of a body indicator monitoring method according to an embodiment of this application;
FIG. 3A is a schematic flowchart of a device selection method according to an embodiment of this application;
FIG. 3B is a schematic flowchart of another device selection method according to an embodiment of this application;
FIG. 4 is a schematic flowchart of another body indicator monitoring method according to an embodiment of this application;
FIG. 5 is a diagram of a scenario in which a user triggers, by performing a body movement 1, blood glucose broadcasting in a normal mode according to an embodiment of this application;
FIG. 6 is a diagram of a scenario in which a user triggers, by performing a body movement 2, blood glucose broadcasting in an alarm mode according to an embodiment of this application;
FIG. 7A to FIG. 7F are some user interfaces that may be used on an electronic device 200 according to an embodiment of this application;
FIG. 8 to FIG. 10 are diagrams of three application scenarios in which a user implements blood glucose broadcasting at home by using a smart home device according to an embodiment of this application;
FIG. 11 is a diagram of a scenario in which a user remotely obtains a body indicator status of a patient by using a mobile phone according to an embodiment of this application;
FIG. 12 is a diagram of a scenario in which a patient implements blood glucose broadcasting by using a headset according to an embodiment of this application;
FIG. 13 is a diagram of a structure of an electronic device 100 (or an electronic device 200) according to an embodiment of this application;
FIG. 14 is a diagram of a structure of an electronic device 300 according to an embodiment of this application;
FIG. 15 is a diagram of a communication module of an electronic device 100 (or an electronic device 200) according to an embodiment of this application;
FIG. 16 is a diagram of a communication module of an electronic device 300 according to an embodiment of this application;
FIG. 17 is a diagram of a hardware structure of an electronic device 700 according to an embodiment of this application;
FIG. 18 is a block diagram of a software structure of an electronic device 700 according to an embodiment of this application; and
FIG. 19 is a diagram of a hardware structure of an electronic device 800 according to an embodiment of this application.

### DESCRIPTION OF EMBODIMENTS

The technical solutions in embodiments of this application are clearly described in detail below with reference to the accompanying drawings. In the descriptions of embodiments of this application, unless otherwise stated, "/" represents the meaning of "or". For example, A/B may represent A or B. "And/or" in the text merely describes an association relationship between associated objects and represents that three relationships may exist. For example, A and/or B may represent the following three cases: Only A exists, both A and B exist, and only B exists. In addition, in the descriptions of embodiments of this application, "a plurality of" means two or more than two.

Hereinafter, the terms "first" and "second" are used only for descriptive purposes, and cannot be understood as indicating or implying relative importance or implicitly indicating a quantity of indicated technical features. Therefore, a feature limited by "first" and "second" may explicitly or implicitly include one or more features. In the descriptions of embodiments of this application, unless otherwise stated, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language such as Java or an extensible markup language (extensible markup language, XML). Interface source code is parsed and rendered on an electronic device, and is finally presented as content that can be recognized by the user. The user interface is usually represented in a form of a graphical user interface (graphic user interface, GUI), and is a user interface that is related to a computer operation and that is displayed in a graphic manner. The user interface may be a visual interface element such as a text, an icon, a button, a menu, a tab, a text box, a dialog box, a status bar, a navigation bar, or a Widget that is displayed on a display of the electronic device.

An embodiment of this application provides a body indicator monitoring method. The method includes: A first device sends first information to a third device based on a first operation; a third device sends obtained data of a first body indicator to a second device after receiving the first information; and the second device outputs the value of the first body indicator, or outputs alarm information when the value of the first body indicator exceeds a first range.

Both the first device and the second device may include one or more devices, for example, a personal device such as a mobile phone, a watch, a band, a headset, a tablet, or a computer of a user, an indoor smart home device such as a smart television, a smart speaker, or a smart display when the user is home, and an in-vehicle terminal in a vehicle.

The third device is a device worn on a patient, and is configured to obtain a value of a body indicator of the patient in real time. For example, when the third device is a CGM device, the first body indicator may be blood glucose; when the third device is a CKM device, the first body indicator may be blood ketone; when the third device is a CKM device, the first body indicator may be lactate; or when the third device is an electrocardiogram patch, the first body indicator may be an electrocardiogram signal.

A wearing position of the third device is usually special, for example, the third device is worn on an upper arm, chest, or abdomen. Generally, the user cannot directly view, only by using the third device, the value that is of the first body indicator and that is obtained by the third device, or an operation performed on the third device is not sufficiently convenient. Therefore, the first device and the second device need to be used to conveniently monitor the first body indicator of the patient by the user.

For ease of differentiation, in the following, a person wearing the third device is referred to as a patient and a person using the first device and the second device is referred to as a user. It should be understood that the user using the first device and the user using the second device may be a same person, or may be different persons. In addition, the patient and the user may be a same person, or may be different persons. This is not limited in this embodiment of this application.

For example, the first device may establish a communication connection to the third device worn by the patient. Specifically, the communication connection may be a wired connection or a wireless connection. The wireless connection may be a short-distance connection such as a high-fidelity wireless communication (wireless fidelity, Wi-Fi) connection, a Bluetooth connection, an infrared connection, an NFC connection, a ZigBee connection, or a human body communication connection, or may be a long-distance connection. The long-distance connection includes but is not limited to a long-distance connection of a mobile network based on 2G, 3G, 4G, 5G, and subsequent standard protocols, or the long-distance connection may be a long-distance connection established after login in to a same account or after login in to a same account group.

For example, using an example in which the first device is a device that establishes a Bluetooth connection to the third device, when the first device receives a user operation that the user requests to monitor a body indicator, the first device may send, by using the Bluetooth connection, an instruction to the third device, to request to obtain a value that is of the body indicator and that is obtained by the third device.

It may be understood that a status of connection between the second device and the third device is similar to that of connection between the first device and the third device, and details are not described herein again.

In addition, the first device and the second device may be a same device, or may be different devices. If the second device and the first device are a same device, when the user acts on the first device to initiate a blood glucose monitoring requirement to the third device, the user may view, by using the first device on which the user currently acts, a body indicator status that is of the patient and that is monitored by the third device. If the second device and the first device are different devices, when the user acts on the first device to initiate a body indicator monitoring requirement to the third device, the user may view, by using the second device, the body indicator status monitored by the third device.

The second device may output, in a manner of displaying, voice broadcast, vibration, or the like, the value that is of the body indicator and that is obtained by the third device. In addition, the second device may be a device determined from a plurality of devices. For details about device selection, refer to the subsequent embodiments. Details are not described herein.

In conclusion, based on the body indicator monitoring method provided in this embodiment of this application, considering that the device configured to monitor the body indicator is not convenient to operate when being worn on the patient, the user can obtain, by using another device, the value that is of the body indicator and that is obtained by the device without directly touching or approaching the device, and monitor a body status of the patient, thereby facilitating user operations. In addition, the body status of the patient may be monitored by outputting a body indicator value of the patient or outputting alarm information when the body indicator of the patient is abnormal. This meets a requirement of the user for monitoring a plurality of body indicators, and improves user experience of monitoring body indicators.

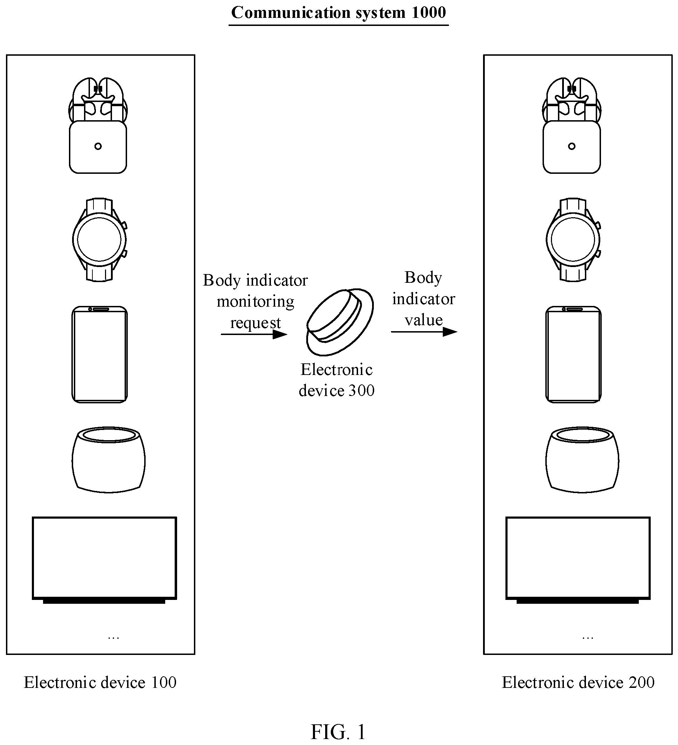

**FIG. 1** **is a diagram of a communication system 1000 according to an embodiment of this application.**

As shown in FIG. 1, the communication system 1000 may include an electronic device 100, an electronic device 200, and an electronic device 300. Both the electronic device 100 and the electronic device 200 may include a plurality of devices, for example, a mobile phone, a watch, a band, a headset, a tablet, a computer, a smart speaker, a smart television, a smart display, and an in-vehicle terminal.

The electronic device 100 may be configured to: receive a user operation that a user requests to broadcast blood glucose, generate a body indicator monitoring request in response to the user operation, and send the body indicator monitoring request to the electronic device 300. Alternatively, the electronic device 100 may be configured to: monitor a body indicator (referred to as a second body indicator below) related to a body indicator monitored by the electronic device 300, generate a body indicator monitoring request when the electronic device 100 determines that a value of the second body indicator exceeds a preset range, and send the body indicator monitoring request to the electronic device 300. Alternatively, after receiving a specified movement of the user, the electronic device 100 may generate a body indicator monitoring request, and send the body indicator monitoring request to the electronic device 300.

The electronic device 300 may be a monitoring device worn on a patient. The electronic device 300 may be configured to obtain a value of a body indicator of the user in real time, for example, a blood glucose value, a blood ketone value, a lactate value, or an electrocardiogram signal. Using an example in which the electronic device 300 is a CGM device, the electronic device 300 may implant a biosensor (a microneedle sensor) under the patient's skin to be in contact with interstitial fluid, to further determine glucose concentration in the interstitial fluid, and then obtain a blood glucose value by compensating for a delay between interstitial fluid glucose and blood glucose.

In addition, after obtaining the body indicator monitoring request, the electronic device 300 may be configured to send a body indicator value obtained by the electronic device 300 to the electronic device 200 based on the body indicator monitoring request.

The electronic device 200 may be configured to: output the body indicator value obtained by the electronic device 300, or output alarm information when the body indicator value obtained by the electronic device 300 exceeds a preset range. The electronic device 200 may output the value of the body indicator and the alarm information in a manner of displaying, voice broadcast, vibration, or the like.

A communication connection may be established between the electronic device 100 and the electronic device 300, and a communication connection may be established between the electronic device 200 and the electronic device 300. For specific descriptions of the communication connection, refer to the foregoing content. Details are not described herein again.

It may be understood that the communication system 1000 may alternatively include more or fewer devices. This is not limited in this embodiment of this application. For example, the communication system 1000 may further include an electronic device 400. The electronic device 100 may send the body indicator monitoring request to the electronic device 400, and then the electronic device 400 sends the body indicator monitoring request to the electronic device 300.

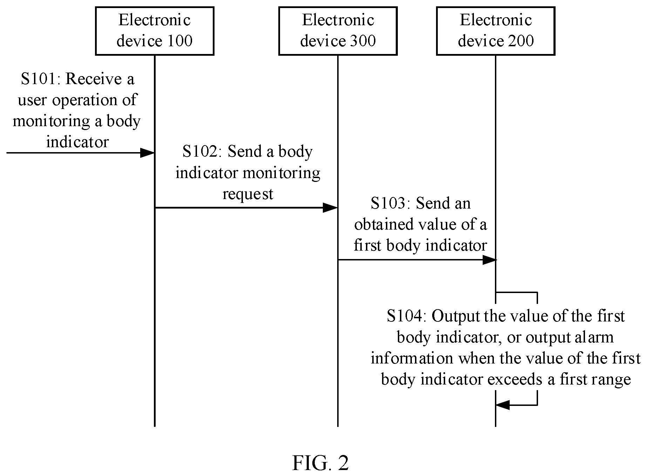

**FIG. 2** **is a schematic flowchart of a body indicator monitoring method according to an embodiment of this application.**

As shown in FIG. 2, the body indicator monitoring method relates to the electronic device 100, the electronic device 200, and the electronic device 300 in the communication system 1000, and may specifically include the following steps.

**S101:** The electronic device 100 receives a user operation of monitoring a body indicator.

For example, the user operation may be a voice command acting on the electronic device 100, or the user operation may be a touch operation performed on a touchscreen of the electronic device 100, or the user operation may be a specified body movement received by the electronic device 100. The electronic device 100 may capture the specified body movement of the user by using a sensor such as an acceleration sensor or a gyroscope sensor. Alternatively, the user operation may be a specified posture (for example, a first posture) made by the user. The electronic device 100 may obtain, by using a camera, the specified posture made by the user.

It may be understood that a form of the user operation is not limited in this embodiment of this application.

**S102:** The electronic device 100 sends a body indicator monitoring request to the electronic device 300.

In response to the user operation in step S101, the electronic device 100 may generate the body indicator monitoring request, and send the body indicator monitoring request to the electronic device 300. Correspondingly, the electronic device 300 receives the body indicator monitoring request sent by the electronic device 100.

It may be understood that the electronic device 100 may send the body indicator monitoring request to the electronic device 200 by using another device. For example, the electronic device 100 may first send the body indicator monitoring request to the electronic device 200, and then the electronic device 200 sends the body indicator monitoring request to the electronic device 300.

In this embodiment of this application, the body indicator monitoring request may also be referred to as first information. A subsequent body indicator monitoring request is similar, and details are not described below again.

**S103:** The electronic device 300 sends an obtained value of a first body indicator to the electronic device 200.

The electronic device 300 may obtain a value of a body indicator of a patient in real time. After the electronic device 300 obtains the body indicator monitoring request sent by the electronic device 100, the electronic device 300 may send, to the electronic device 200, the value that is of the body indicator and that is obtained by the electronic device 300. The value that is of the body indicator and that is sent by the electronic device 200 may include the following three cases:
(1) The value of the body indicator may be a value that is of a body indicator and that is currently obtained by the electronic device 300 in real time.

In other words, the electronic device 300 may send, to the electronic device 200 based on a user operation performed by the user on the electronic device 100, the value that is of the body indicator and that is obtained by the electronic device 300 at a current moment, so that the electronic device 200 can immediately obtain the value of the body indicator of the current patient.

(2) The value of the body indicator includes values of the body indicator that are continuously obtained by the electronic device 300 starting from the current moment.

In other words, the electronic device 300 may start to continuously send, to the electronic device 200 based on a user operation performed by the user on the electronic device 100, the values of the body indicator that are obtained by the electronic device 300, so that the electronic device 200 can continuously collect the values of the body indicator that are obtained by the electronic device 300.

(3) The value of the body indicator may be a value that is of the body indicator, that exceeds a preset range, and that is obtained by the electronic device 300 starting from the current moment.

In other words, the electronic device 300 may control, based on a user operation performed by the user on the electronic device 100, the electronic device 300 to monitor a body indicator status of the patient, and send the value of the body indicator to the electronic device 200 when the value of the body indicator of the patient exceeds the preset range, so that the electronic device 200 can obtain the abnormal value of the body indicator of the patient.

It may be understood that the value of the first body indicator may include one or more values. The value may be an actual value of the first body indicator of the user, or may be a processed value of the first body indicator, for example, a difference relative to historical data of the user. In other words, the value may indicate a current actual status of the first body indicator of the user, or indicate a current-historical change status of the first body indicator of the user. Using an example in which the first body indicator is blood glucose, the value may be an actual blood glucose value of the user measured by the electronic device 300, or may be a difference between two consecutive blood glucose values of the user measured by the electronic device 300. A specific meaning of the value of the first body indicator is not limited in this embodiment of this application.

**S104:** The electronic device 200 outputs the value of the first body indicator, or outputs alarm information when the value of the first body indicator exceeds a first range.

The electronic device 200 may output the value of the body indicator or the alarm information in a manner of displaying, voice broadcast, vibration, or the like, to remind the user. In addition, that the electronic device 200 outputs the alarm information may alternatively be a case in which an emergency call is made, a call is made to a family member, or the like. This embodiment of this application imposes no limitation on the alarm information.

With reference to the value that is of the body indicator and that is sent by the electronic device 300 to the electronic device 200 in step S103, it can be learned that the value that is of the body indicator and that is output by the electronic device 200 may include the following three cases:
(1) The value of the body indicator may be a value that is of a body indicator and that is currently obtained by the electronic device 300 in real time.

In other words, after the user initiates a user operation to the electronic device 100, the user can view a current body indicator status of the patient by using the electronic device 200.

(2) The value of the body indicator includes values of the body indicator that are continuously obtained by the electronic device 300 starting from the current moment.

In other words, after the user initiates a user operation to the electronic device 100, the user may continuously monitor the body indicator of the patient, and the user can not only view a current body indicator status of the patient by using the electronic device 200, but also view a body indicator change status of the patient within a period of time by using the electronic device 200.

(3) The value of the body indicator may be a value that is of the body indicator, that exceeds a preset range, and that is obtained by the electronic device 300 starting from the current moment.

In other words, after the user initiates a user operation to the electronic device 100, the user can learn of an abnormal body indicator state of the patient by using the electronic device 200.

It can be learned from the foregoing three cases that, for different values of the body indicator that are output by the electronic device 200, the electronic device 200 may broadcast the body indicator in a plurality of modes, including a normal mode, a monitoring mode, and an alarm mode.

The normal mode may be that the electronic device 200 outputs the value that is of the body indicator and that is currently obtained by the electronic device 300 (that is, Case 1). The monitoring mode may be that the electronic device 200 outputs the values of the body indicator that are continuously obtained by the electronic device 300 from the current moment (that is, Case 2). The alarm mode may be that the electronic device 200 outputs the value that is of the body indicator, that exceeds a threshold, and that is obtained by the electronic device 300 or the alarm information (that is, Case 3).

For example, a specific mode in which the electronic device 200 broadcasts the body indicator may be determined by a user operation performed on the electronic device 100. For example, if the user operation performed on the electronic device 100 is an operation 1, the electronic device 200 may output a value of the body indicator in the normal mode. If the user operation performed on the electronic device 100 is an operation 2, the electronic device 200 may output a value of the body indicator in the monitoring mode. If the user operation performed on the electronic device 100 is an operation 3, the electronic device 200 may output a value of the body indicator or the alarm information in the alarm mode.

In this way, different user operations performed on the electronic device 100 may enable the electronic device 200 to output values of the body indicator in different cases, to meet different body indicator monitoring requirements of the user.

In some implementations, that the electronic device 200 outputs the value of the first body indicator may be that the electronic device 200 displays the value of the first body indicator and/or displays a first body indicator curve, where the first body indicator curve includes a plurality of values of the first body indicator that are obtained by the electronic device 300. Using an example in which the first body indicator is blood glucose, the electronic device 200 may display a blood glucose value and/or a blood glucose curve, to display a blood glucose status of the user from different aspects.

In addition, the electronic device 200 may be a device determined from a plurality of devices. The plurality of devices may include the electronic device 100 and a device that establishes a communication connection to the electronic device 100, for example, a personal device such as a mobile phone, a watch, a band, a headset, a tablet, or a computer, and an indoor smart home device such as a smart television, a smart speaker, or a smart display when the user is home.

For example, a device most suitable for outputting the value of the body indicator of the patient or the alarm information may be determined from the plurality of devices based on priorities of device types.

Assuming that the device types are a watch/band, a headset, a tablet/computer, a smart home, and a mobile phone in descending order of the priorities, a device that is currently available to the user and that has a highest priority may be selected as the electronic device that outputs the value of the body indicator or the alarm information. For example, if the plurality of devices include a watch/band, the value of the body indicator of the patient or the alarm information may be directly output by using the watch/band. If the plurality of devices do not include a watch/band, whether the plurality of devices include a headset may continue to be searched. If the plurality of devices include a headset, the value of the body indicator of the patient or the alarm information is output by using the headset, and so on.

In some implementations, after obtaining the value of the body indicator, the electronic device 200 may alternatively upload the value of the body indicator to a cloud server, and another user that is far away from the patient may remotely monitor a body indicator status of the patient by obtaining the value that is of the body indicator and that is stored in the cloud server.

It may be understood that after the electronic device 300 obtains the value of the body indicator, the electronic device 300 may alternatively upload the value of the body indicator to the cloud server. This is not limited in this embodiment of this application.

In some implementations, the electronic device 100 and the electronic device 300 may be a same device. In this way, the electronic device 300 may directly receive the user operation to trigger monitoring on the body indicator, and send the obtained value of the body indicator to the electronic device 200, and the electronic device 200 outputs the value of the body indicator, or outputs the alarm information when the value of the body indicator exceeds the first range.

In some implementations, the electronic device 200 may broadcast the body indicator in different modes. Therefore, for body indicator broadcasting in different modes, different device selection methods may be used to determine a device that broadcasts the body indicator.

In the normal mode or the monitoring mode, the user needs to know the current value of the body indicator of the patient. Therefore, the device that outputs the body indicator may be determined by using privacy of information presentation as a reference factor.

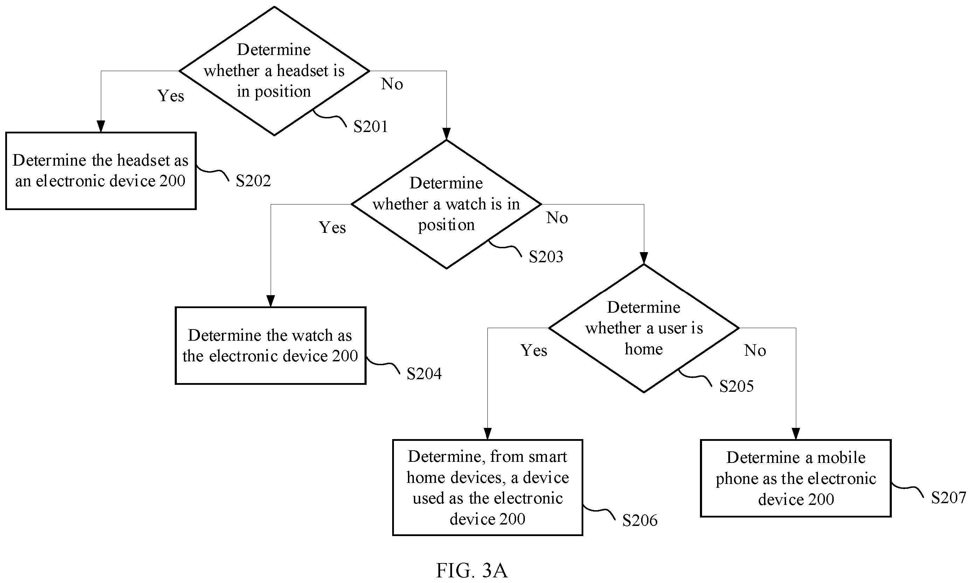

**For example,** **FIG. 3A** **is a schematic flowchart of a device selection method according to an embodiment of this application.**

**S201:** Determine whether a headset is in position.

Whether the headset is in position may be whether the electronic device 100 and the device that establishes a communication connection to the electronic device 100 include the headset.

Further, whether the headset is in position may further include: whether the user wears the headset.

If the headset is in position, it indicates that the user can listen to the value that is of the body indicator and that is broadcast by the headset. Therefore, if the headset is in position, step S202 is performed; and otherwise, step S203 is performed.

**S202:** Determine the headset as the electronic device 200.

In other words, if the electronic device 100 and the device that establishes a communication connection to the electronic device 100 include the headset, the headset may be preferentially selected to output the value of the body indicator of the patient.

This is because sound broadcast by the headset is usually heard only by the user. Broadcasting the body indicator of the patient by using the headset can improve privacy of broadcasting the body indicator, and prevent another person other than the user from learning the body indicator status of the patient.

**S203:** Determine whether a watch is in position.

Whether the watch in position may be whether the electronic device 100 and the device that establishes a communication connection to the electronic device 100 include the watch.

Further, whether the watch is in position may further include: whether the user wears the watch.

If the watch is in position, it indicates that the user can view the value of the body indicator of the patient by using the watch. Therefore, if the watch is in position, step S204 is performed; and otherwise, step S205 is performed.

**S204:** Determine the watch as the electronic device 200.

In other words, if the electronic device 100 and the device that establishes a communication connection to the electronic device 100 do not include a headset but include a watch, the value of the body indicator of the patient may be output by using the watch.

This is because information displayed by the watch is usually visible only to the user and a person close to the user. Displaying the value of the body indicator of the patient by using the watch can also ensure privacy of broadcasting the body indicator, and prevent, to the greatest extent, another person other than the user from learning the body indicator status of the patient.

It may be understood that, for a band that is also a wearable device, the watch in steps S203 and S204 may alternatively be changed to a band. This is not limited in this embodiment of this application.

**S205:** Determine whether the user is home.

If the user does not use a device such as a headset or a watch, it may be further determined whether the user is home, and the body indicator is further broadcast with reference to a smart home device at the user's home.

For example, current position information of the user may be obtained by using a device carried by the user, for example, a mobile phone, and whether the user is home is determined by using the location information. Alternatively, a surveillance camera at home may be used to identify family members at home, to determine whether the user is home. A manner of determining whether the user is home is not limited in this embodiment of this application.

If the user is home, step S206 is performed; and otherwise, step S207 is performed.

**S206:** Determine, from smart home devices, a device used as the electronic device 200.

If the user is home, and the electronic device 100 and the device that establishes a communication connection to the electronic device 100 include the smart home devices, the device configured to output the value of the body indicator may be determined from the smart home devices.

Because there are usually a plurality of smart home devices at home, a proper device may be further selected from the plurality of smart home devices as the electronic device 200 to output the value of the body indicator of the patient.

For example, the user may preset priorities of the smart home devices included at home, and when the value of the body indicator needs to be broadcast, a device with a highest priority is selected as the electronic device 200.

Further, the user may further classify the smart home devices into graphic display devices and voice broadcast devices. When the value of the body indicator needs to be broadcast, the user may first select whether to output the value of the body indicator by using a graphic display device or output the value of the body indicator by using a voice broadcast device, and then determine, from the type of device selected by the user, a device with a highest priority as the electronic device 200.

In some implementations, the electronic device 200 may alternatively be determined from the smart home devices based on a position of the user, for example, a room in which the user is located.

S207: Determine a mobile phone as the electronic device 200.

Generally, the mobile phone is a necessary device of the user. If the user does not use a device such as a headset or a watch, and is not home, the value of the body indicator of the patient may be output by using the mobile phone in the plurality of devices.

It can be learned from steps S201 to S207 that, if the user triggers the electronic device 200 to output the value that is of the body indicator and that is obtained by the electronic device 300 in real time, the electronic device 200 may be determined based on privacy when the device outputs information, and a device with best privacy effect is selected as the electronic device 200, to ensure, to the greatest extent, privacy when the body indicator of the patient is broadcast.

It may be understood that steps S201 to S207 may be performed by the electronic device 100, or may be performed by the electronic device 300, or may be performed by a device with a strongest computing capability in a plurality of electronic devices. The plurality of electronic devices may include some or all of the following: the electronic device 100, the electronic device 300, the device that establishes a communication connection to the electronic device 100, and a device that establishes a communication connection to the electronic device 300. This is not limited in this embodiment of this application.

In another embodiment of this application, alternatively, step S205 may be performed first, and then step S203 is performed. In this way, when the user triggers to broadcast the current value of the body indicator of the patient, the device configured to broadcast the body indicator may be selected in an order of the headset, the smart home device, the watch, and the mobile phone of the user.

In addition, in the alarm mode, the user needs to obtain an abnormal body indicator state of the patient in a timely manner when the body indicator of the patient is abnormal. Therefore, the device that outputs the alarm information may be determined by using timeliness of information presentation as a reference factor.

In a specific implementation, if the electronic device 300 obtains an abnormal value of the body indicator of the patient, a device currently used by the user may be found, and the value of the body indicator of the patient is output by using the device currently used by the user.

Further, optionally, to prevent the user from missing the body indicator information, the value of the body indicator of the patient may be output by using a plurality of devices.

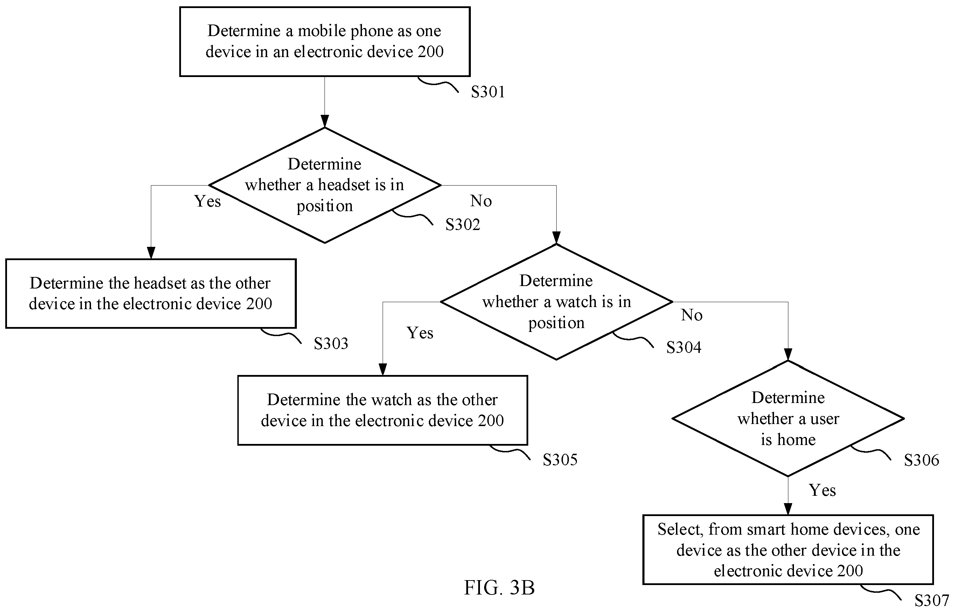

**For example,** **FIG. 3B** **is a schematic flowchart of another device selection method according to an embodiment of this application by using an example in which the electronic device 200 includes two devices.**

**S301:** Determine a mobile phone as one device in the electronic device 200.

In other words, when the body indicator of the patient is abnormal, the alarm information may be directly output by using the mobile phone of the user, to remind the user of the abnormal body indicator state of the patient.

This is because the mobile phone is the most frequently used and necessary terminal device of people, and when the alarm information is output on the mobile phone, the user can be prevented from missing the information.

In some implementations, before step S301, whether the mobile phone is in a screen-on state may be further determined first. If the mobile phone is in the screen-on state, a probability that the user is currently using the mobile phone is high. Therefore, the alarm information may be output by using the mobile phone, and the user is reminded in a timely manner in a process in which the user uses the mobile phone.

**S302:** Determine whether a headset is in position.

If the headset is in position, step S303 is performed; and otherwise, step S304 is performed.

**S303:** Determine the headset as the other device in the electronic device 200.

If the headset is in position, it indicates that there is a high probability that the user is currently listening to audio by using the headset. Therefore, with reference to the abnormal value that is of the body indicator of the patient and that is output on the mobile phone in combination with the abnormal value that is of the body indicator and that is output by using the headset, it can be further ensured that the user learns of the abnormal body indicator state of the patient in a timely manner.

**S304:** Determine whether a watch is in position.

If the watch is in position, step S305 is performed; and otherwise, step S306 is performed.

**S305:** Determine the watch as the other device in the electronic device 200.

If the user does not use the headset, the abnormal value of the body indicator of the patient may be output by using the watch currently worn by the user, to ensure, to the greatest extent, that the user can learn of the abnormal body indicator state of the patient in a timely manner, and prevent the user from missing the body indicator information.

S306: Determine whether the user is home.

If the user is home, step S307 is performed.

S307: Determine, from smart home devices, one device as the other device in the electronic device 200.

In some implementations, a smart home device currently used by the user may be used to determine the device configured to output the value of the body indicator. For example, if a smart display is in a screen-on state, the user may be watching a video by using the smart display, and the current abnormal value of the body indicator of the patient may be displayed on the smart display. For another example, if a smart speaker is in a working state, the user may be listening to music played by the smart speaker, and the current abnormal value of the body indicator of the patient may be broadcast by using the smart speaker.

In some other implementations, the device used by the user to output the value of the body indicator may alternatively be determined with reference to a distance between a smart home and the user. For example, a smart home device that is currently closest to the user may be selected as the other device in the electronic device 200. In this way, it can be ensured, to the greatest extent, that the user views the abnormal body indicator state of the patient in a timely manner.

It may be understood that the device may alternatively be selected from the smart home devices in another manner. For example, priorities of the smart home devices are set in advance, and the device is selected based on the priorities. This is not limited in this embodiment of this application.

For specific content that is not described in detail in steps S301 to S307, refer to related content in the foregoing steps S201 to S207. Details are not described herein again.

It can be learned from steps S201 to S207 that, if the user triggers the electronic device 200 to output the alarm information when the body indicator of the patient is abnormal, the device may be determined based on timeliness of receiving the information by the user, to ensure that the user obtains the alarm information in a timely manner. In addition, the value of the body indicator may be output by using a plurality of devices, to prevent the user from missing the alarm information.

In conclusion, if the user triggers body indicator broadcasting in the normal mode or the monitoring mode, the device configured to broadcast the body indicator may be determined based on the device selection method shown in FIG. 3A. If the user triggers body indicator broadcasting in the alarm mode, the device configured to broadcast the body indicator may be determined based on the device selection method shown in FIG. 3B.

It may be understood that the electronic device 200 may alternatively be determined based on a same device selection method for body indicator broadcasting in different modes, or the electronic device 200 may alternatively be determined based on another device selection method. This is not limited in this embodiment of this application.

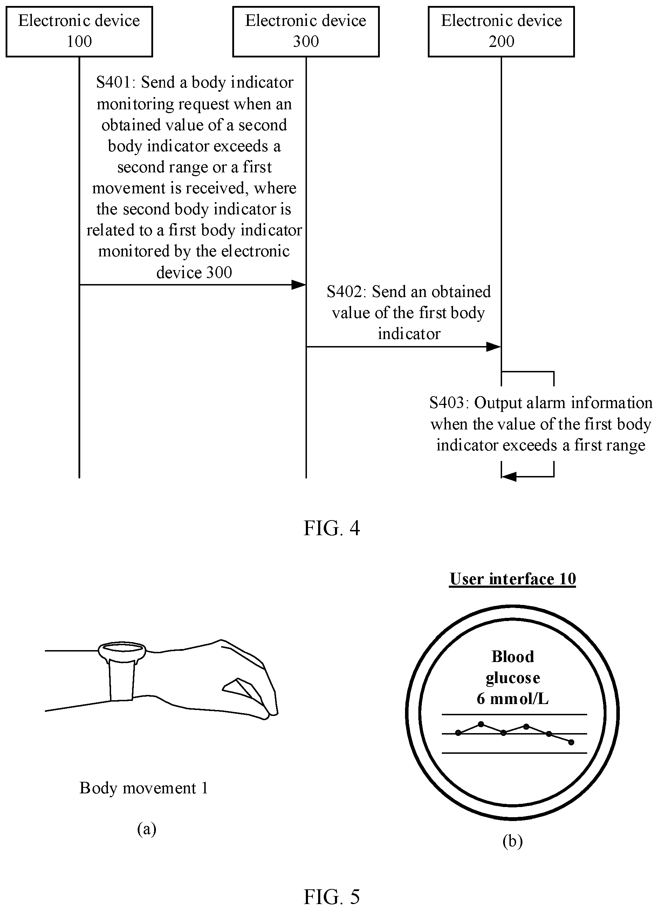

**FIG. 4** **is a schematic flowchart of another body indicator monitoring method according to an embodiment of this application.**

As shown in FIG. 4, the body indicator monitoring method relates to the electronic device 100, the electronic device 200, and the electronic device 300 in the communication system 1000, and may specifically include the following steps.

S401: The electronic device 100 sends a body indicator monitoring request to the electronic device 300 when an obtained value of a second body indicator exceeds a second range or a first movement is received, where the second body indicator is related to a first body indicator monitored by the electronic device 300.

The electronic device 100 and the electronic device 300 may be devices used by a same user.

In this embodiment of this application, the electronic device 100 may have the following two functions:
(1) The electronic device 100 may monitor a body indicator of a user.

The electronic device 100 may be configured to obtain the second body indicator of the user, and the electronic device 300 may be configured to obtain the first body indicator of the user. The second body indicator is related to the first body indicator.

For example, the electronic device 100 may be a wearable device such as a headset or a watch, the electronic device 300 may be a CGM device, the electronic device 100 may monitor a heart rate of the user, and the electronic device 300 may monitor blood glucose of the user. If the blood glucose of the user is high for a long time, the heart rate of the user may be accelerated.

When the second body indicator monitored by the electronic device 100 is abnormal, the blood glucose of the user may also be abnormal. Therefore, the electronic device 100 may initiate a monitoring request for the first body indicator to the electronic device 300 when the value that is of the second body indicator and that is obtained by the electronic device 100 is abnormal.

(2) The electronic device 100 may receive a movement of the user.

Considering that the user wearing the electronic device 300 is generally a patient with a problem in a body indicator, in daily life of the user, it is particularly necessary to pay attention to whether various movements performed by the user cause an abnormal change in the body indicator of the user. Therefore, the electronic device 100 may initiate the monitoring request for the first body indicator to the electronic device 300 when receiving some specified movements performed by the user.

For example, the first movement may be a movement such as squatting, falling, jumping, or running. The first movement is not limited in this embodiment of this application. The electronic device 100 may receive the first movement based on a component such as a gyroscope sensor or an acceleration sensor.

S402: The electronic device 300 sends an obtained value of the first body indicator to the electronic device 200.

After the electronic device 300 obtains the body indicator monitoring request sent by the electronic device 100, the electronic device 300 may send, to the electronic device 200, the value that is of the body indicator and that is obtained by the electronic device 300.

S403: The electronic device 200 outputs alarm information when the value of the first body indicator exceeds a first range.

If the value that is of the first body indicator and that is obtained by the electronic device 300 exceeds a normal range of the first body indicator, it indicates that the current body indicator of the user is abnormal, and the electronic device 200 may output the alarm information, to remind the user of the current body abnormal status in a timely manner.

It can be learned from steps S401 to S403 that, when another body indicator is abnormal, or when the user performs some movements, the user may link the electronic device 300 to measure the body indicator of the user, to pay attention, in a timely manner, to the abnormal value obtained by the electronic device 300, and when the user does not realize a body abnormal status, the user is reminded in a timely manner, so that the user can take measures in a timely manner to ensure a normal body status of the user.

It may be understood that, for specific content that is not mentioned in steps S401 to S403, for example, a plurality of cases in which the electronic device 300 obtains the value of the first body indicator, and selection of the electronic device 200, refer to related content in the foregoing FIG. 2, FIG. 3A, and FIG. 3B. Details are not described herein again.

**The following describes in detail a plurality of application scenarios of a body indicator monitoring method according to an embodiment of this application.**
**(1)** The user implements body indicator monitoring by using an intelligent wearable device such as a watch or a band.

In this application scenario, the electronic device 100 may be an intelligent wearable device such as a watch or a band.

Because the intelligent wearable device is equipped with a sensor that can detect a body movement of the user, such as an acceleration sensor or a gyroscope sensor, the user operation in step S101 may be the body movement of the user. In this way, the user only needs to wear a wearable device such as a watch or a band, and can obtain, by performing a simple body movement, body indicator information obtained by the device configured to monitor the body indicator, thereby facilitating an operation of the user.

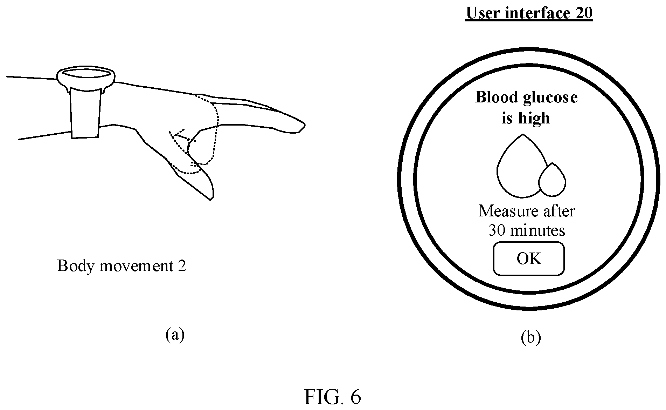

For example, by using an example in which the electronic device 100 and the electronic device 200 are a same device, the electronic device 300 is a CGM device, and a body indicator monitored by the electronic device 300 is blood glucose, FIG. 5 and FIG. 6 are diagrams of scenarios in which a user triggers blood glucose broadcasting by performing a body movement.

FIG. 5 is a diagram of a scenario in which a user triggers, by performing a body movement 1, blood glucose broadcasting in a normal mode. (a) in FIG. 5 shows the body movement 1 in which the user pinches and rubs a finger, and (b) in FIG. 5 shows a user interface 10 displayed when the electronic device 200 outputs a blood glucose value. The user interface 10 may be configured to display a current blood glucose value that is of the patient and that is obtained by the electronic device 300 under triggering of the body movement of the user.

Optionally, the user interface 10 may be further configured to display a blood glucose curve. The blood glucose curve may include a current blood glucose value and blood glucose values that are historically measured by the electronic device 300 of the patient.

It may be understood that, in addition to displaying the current blood glucose value of the patient, the electronic device 200 may further input the blood glucose value of the patient in a voice broadcasting manner.

FIG. 6 is a diagram of a scenario in which a user triggers, by performing a body movement 2, blood glucose broadcasting in an alarm mode. (a) in FIG. 6 shows the body movement 2 in which the user clenches a fist and then releases the fist, and (b) in FIG. 6 shows a user interface 20 displayed when the electronic device 200 outputs abnormal blood glucose. Alarm information displayed on the user interface 20 may prompt the user that the current blood glucose value of the patient is high.

Optionally, the alarm information may further include the current abnormal blood glucose value of the patient. In addition, the electronic device 200 may broadcast the abnormal blood glucose value of the patient by using voice.

In some implementations, after receiving the body movement 2, the electronic device 200 may further output prompt information, where the prompt information prompts the user that blood glucose broadcasting in an alarm mode has been enabled on the electronic device 200 currently. In this way, the user can learn, based on the prompt information, that the electronic device 200 has enabled blood glucose broadcasting in the alarm mode currently. In addition, if the electronic device 200 does not broadcast a blood glucose status currently, the user may learn that current blood glucose of the patient is normal, instead of because the blood glucose of the patient is not identified or the body movement 2 of the user is not received. For example, the electronic device 200 may output the prompt information in a manner of displaying, voice broadcast, vibration, or the like. A manner of outputting the prompt information is not limited in this embodiment of this application.

It may be understood that FIG. 5 and FIG. 6 are merely examples. In this embodiment of this application, the electronic device 100 (or the electronic device 200) may be further triggered, by using another body movement, to output the blood glucose value or the alarm information.

It can be learned from FIG. 5 and FIG. 6 that the user may trigger, by performing different body movements, the watch or the band to broadcast blood glucose statuses in different modes, to meet different blood glucose monitoring requirements of the user.

It should be noted that, if the electronic device 300 is configured to monitor another body indicator, for example, blood ketone or lactate, a scenario in which the electronic device 100 triggers, by using different body movements, body indicator broadcasting in different modes is similar to that of the foregoing FIG. 5 and FIG. 6. This is not described in detail again in this embodiment of this application.

In some implementations, detecting the body movement of the user only by using the electronic device 100 may be inaccurate. Therefore, when the user using the electronic device 100 and the patient wearing the electronic device 300 are a same person, whether the user has a body indicator monitoring requirement may be further determined with reference to a body movement received by the electronic device 300, to provide a more accurate body indicator monitoring service for the user.

In this case, a sensor configured to detect a body movement of the user such as an acceleration sensor or a gyroscope sensor may be preset in the electronic device 300, to detect the body movement of the user.

In a specific implementation, when the electronic device 100 receives a first body movement and the electronic device 300 receives the same body movement, the electronic device 200 may output the value that is of the body indicator and that is obtained by the electronic device 300 or the alarm information. In other words, if both the electronic device 100 and the electronic device 300 can receive a user operation initiated by the user for blood glucose broadcasting, it indicates that the user indeed has a body indicator monitoring request currently, and the electronic device 200 may output the value of the body indicator or the alarm information. In this way, the operation of the user can be identified collaboratively by using a plurality of devices, to avoid misidentification, thereby implementing accurate body indicator broadcasting.

It may be understood that, in addition to the body movement of the user, the user operation received by the electronic device 100 may alternatively be a physical operation performed by the user on a button in the electronic device 100, a touch operation performed on a touchscreen of the electronic device 100, a movement of shaking an arm wearing the electronic device 100, or the like. This operation is not limited in this embodiment of this application.

In some implementations, because a wearable device such as a watch or a band may further measure other body indicators such as a heart rate, blood oxygen, a body temperature, and a step count, before the electronic device 200 outputs the alarm information, whether the electronic device 200 outputs the alarm information may be further determined with reference to another body indicator that is related to the body indicator monitored by the electronic device 300 and that is of the patient. For example, if the body indicator monitored by the electronic device 300 is blood glucose, another body indicator related to the blood glucose may be a heart rate.

In a specific implementation, after the user triggers body indicator broadcasting in the alarm mode, if the electronic device 300 obtains an abnormal value of the body indicator, the electronic device 100 or the electronic device 200 may further obtain another body indicator related to the body indicator of the patient, and determine whether the another body indicator exceeds a preset range, for example, a second range. If the another body indicator exceeds the preset range, the electronic device 200 may output the alarm information; and otherwise, the electronic device 200 may not output the alarm information.

In this way, the body status of the patient may be evaluated with reference to a plurality of factors, to avoid a false alarm of the electronic device 200, reduce a quantity of alarms of the electronic device 200, and improve accuracy of monitoring the body indicator of the patient.

It may be understood that, although detailed content of the body indicator monitoring method is described by using a band or a watch as an example in this application scenario, in this embodiment of this application, a device that can receive a body of the user or that can measure a physiological indicator of the user should fall within the protection scope of this application. Example device types in this application scenario do not constitute a limitation on this embodiment of this application. This is similar in the following application scenarios, and details are not described in this embodiment of this application.

**(2)** The user implements body indicator monitoring by using a mobile phone.

In this application scenario, the electronic device 100 and/or the electronic device 200 may be a mobile phone. In this way, the user may trigger body indicator broadcasting by performing an operation on the mobile phone, and/or the user may learn a body indicator status of the patient on the mobile phone.

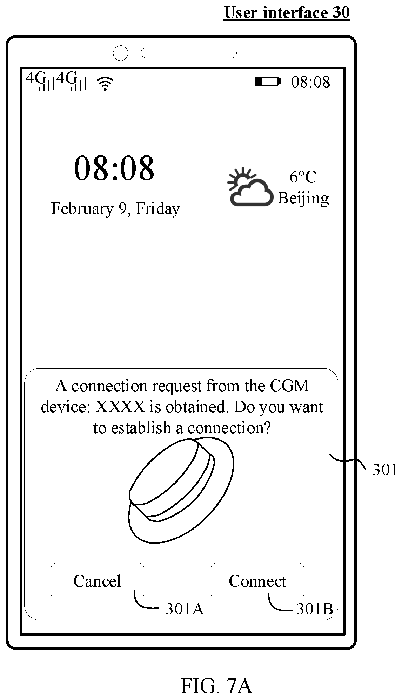

For example, by using an example in which the electronic device 200 is a mobile phone, the electronic device 300 is a CGM device, and the body indicator monitored by the electronic device 300 is blood glucose, FIG. 7A to FIG. 7F show some user interfaces that may be used on the electronic device 200.

FIG. 7A shows a user interface 30 displayed when the electronic device 200 receives a connection request of the electronic device 300.

As shown in FIG. 7A, the user interface 30 may include a connection window 301. The connection window 301 may be used to prompt the user that the CGM device requests to establish a connection. The connection window 301 may include a cancel option 301A and a connection option 301B. The cancel option 301A may be used to reject to establish a connection to the CGM device (that is, the electronic device 300), and the connection option 301B may be used to agree to establish a connection to the electronic device 300.

For example, if the electronic device 200 receives a user operation performed by the user on the connection option 301B, for example, a tap operation, the electronic device 200 establishes a communication connection to the electronic device 300 in response to the operation. Then, the electronic device 200 may obtain a blood glucose value obtained by the electronic device 300, and output the blood glucose value or alarm information.

It may be understood that after Bluetooth is enabled and the patient wears the CGM device for the first time, the electronic device 200 may display the user interface 30 shown in FIG. 7A Then, when the electronic device 200 establishes a connection to the CGM device again, the connection window 301 in the user interface 30 may not need to be displayed, to implement seamless connection between the electronic device 200 and the CGM device, thereby reducing user operations.

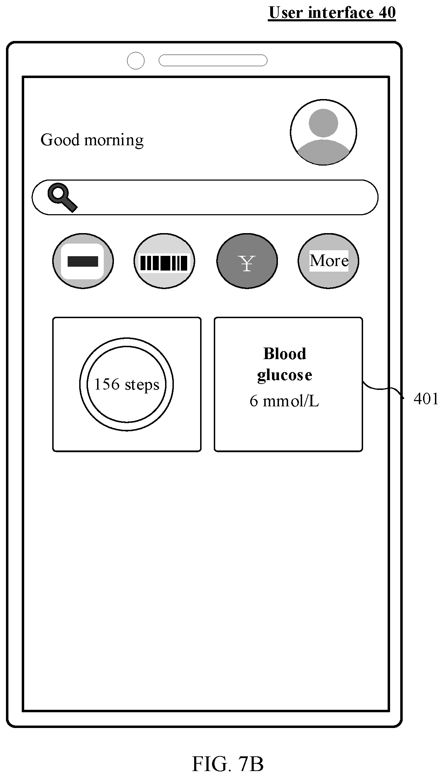

FIG. 7B shows a user interface 40 for the electronic device 200 to display a blood glucose value on a leftmost screen.

The leftmost screen may be a page displayed after the electronic device 200 receives a left flick operation of the user when displaying a desktop home screen. The leftmost screen may be used to display a plurality of third-party plug-in functions or service cards, to provide a service for the user to use some functions of an application without starting the application.

As shown in FIG. 7B, the user interface 40 may include a card 401. The card 401 may be configured to display the blood glucose value obtained by the electronic device 300. In this way, when the user needs to view a blood glucose status of the patient, the user may switch to the leftmost screen at any moment for viewing.

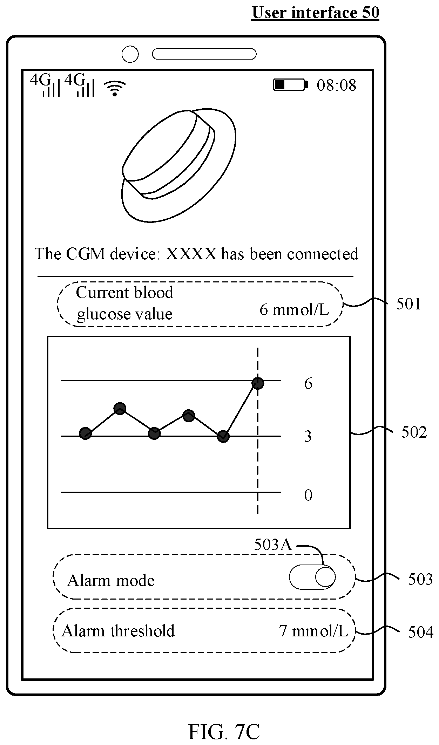

For example, when the electronic device 100 receives a user operation performed by the user on the card 401, for example, a tap operation, the electronic device 100 may display a user interface 50 shown in FIG. 7C in response to the operation. The user interface 50 may be configured to display details of blood glucose of the patient.

As shown in FIG. 7C, the user interface 50 may include: a current blood glucose value 501, a blood glucose curve 502, an alarm mode 503, and an alarm threshold 504.

The current blood glucose value 501 is used to display a blood glucose value that is of the patient and that is obtained by the electronic device 300 currently, for example, 6 mmol/L. The blood glucose curve 502 may be used to display a curve obtained by connecting blood glucose values obtained by the electronic device 300 at a plurality of moments. The alarm mode 503 may be used to trigger the electronic device 200 to enable or disable blood glucose broadcasting in the alarm mode. The alarm mode 503 may include a switch 503A. If the electronic device 200 receives a user operation performed on the switch 503A, the electronic device 200 may enable blood glucose broadcasting in the alarm mode, that is, output the blood glucose value of the patient only when the blood glucose value of the patient exceeds a threshold. If the electronic device 200 receives, again, a user operation performed on the switch 503A, the electronic device 200 may disable blood glucose broadcasting in the alarm mode, that is, the electronic device 200 does not output the blood glucose value of the patient only when the blood glucose value of the patient exceeds the threshold. The alarm threshold 504 may be used to set a blood glucose threshold in the alarm mode. For example, if the user sets the blood glucose threshold to 7 mmol/L, if the blood glucose value that is of the patient and that is obtained by the electronic device 300 is greater than 7 mmol/L, the electronic device 300 outputs alarm information, to prompt the user that current blood glucose of the patient is abnormal.

It may be understood that the user interface 50 may alternatively display only one of the current blood glucose value 501 and the blood glucose curve 502. This is not limited in this embodiment of this application.

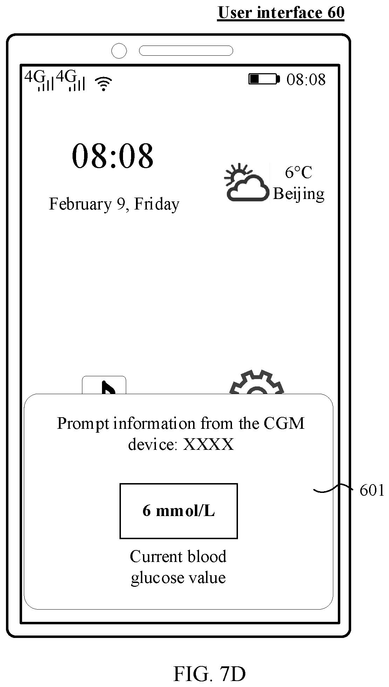

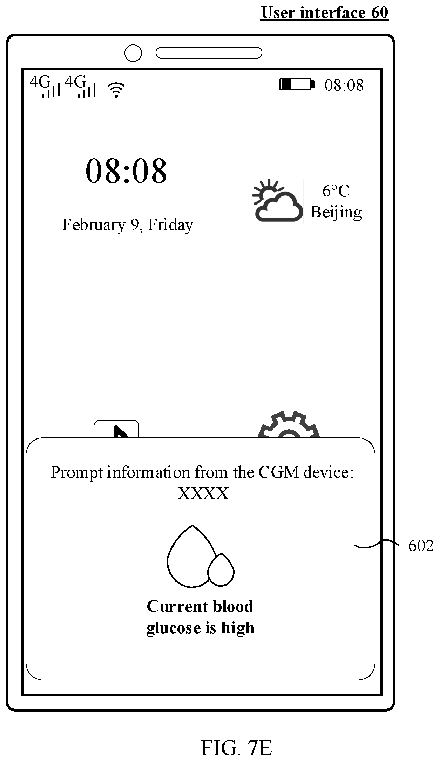

FIG. 7D and FIG. 7E show another user interface 60 for the electronic device 200 to output a blood glucose value respectively in the normal mode and the alarm mode.

As shown in FIG. 7D, the user interface 60 may include prompt information 601. The prompt information 601 may be used to display a current blood glucose value, for example, 6 mmol/L, of the patient to the user when the user triggers blood glucose broadcasting in the normal mode by using the electronic device 100.

As shown in FIG. 7E, the user interface 60 may include prompt information 602. The prompt information 601 may be used to display, to the user, alarm information that the blood glucose of the patient is abnormal, for example, the blood glucose of the patient is high, when the blood glucose value of the patient exceeds the threshold after the user triggers blood glucose broadcasting in the alarm mode by using the electronic device 200. Optionally, the prompt information 602 may include information such as a current blood glucose value and a normal blood glucose range.

In some implementations, the electronic device 100 and the electronic device 200 may be a same device. In this case, the electronic device 100 may trigger voice broadcast by using a voice instruction of the user, and output the current blood glucose value of the patient by using voice. In other words, the user operation in step S101 may be a voice instruction of the user, and in step S104, the electronic device 200 may output the blood glucose value or the alarm information by using voice.

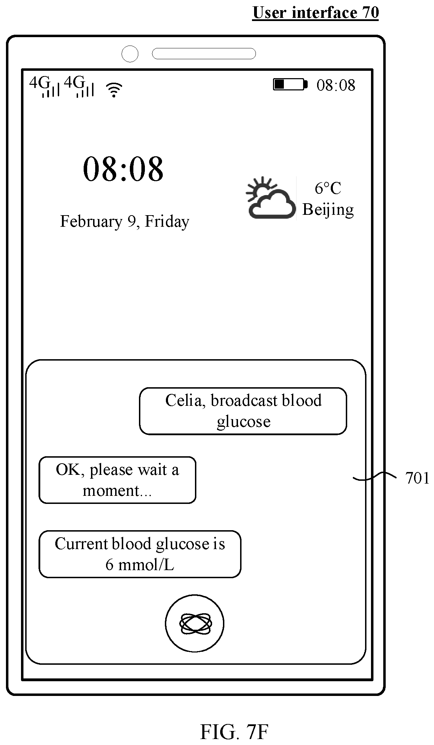

FIG. 7F shows a user interface 70 displayed when the electronic device 100 broadcasts blood glucose by using a voice indication of the user.

As shown in FIG. 7F, the user interface 70 may include a voice prompt window 701. The voice prompt window 701 may be configured to display texts obtained by converting a recognized voice instruction of the user by the electronic device 100, and texts corresponding to voice output by the electronic device 100. It can be learned from the voice prompt window 701 that the voice instruction of the user includes "Celia, broadcast blood glucose", and the voice output by the electronic device 200 includes "OK, please wait a moment...", and "Current blood glucose is 6 mmol/L".

It can be learned from FIG. 7F that the user may trigger, by using voice, the electronic device to broadcast the blood glucose of the patient, to help the user quickly and conveniently view the blood glucose status of the patient.

(3) The user implements body indicator monitoring at home by using a smart home device.

In this application scenario, the electronic device 100 and/or the electronic device 200 may be a smart home device.

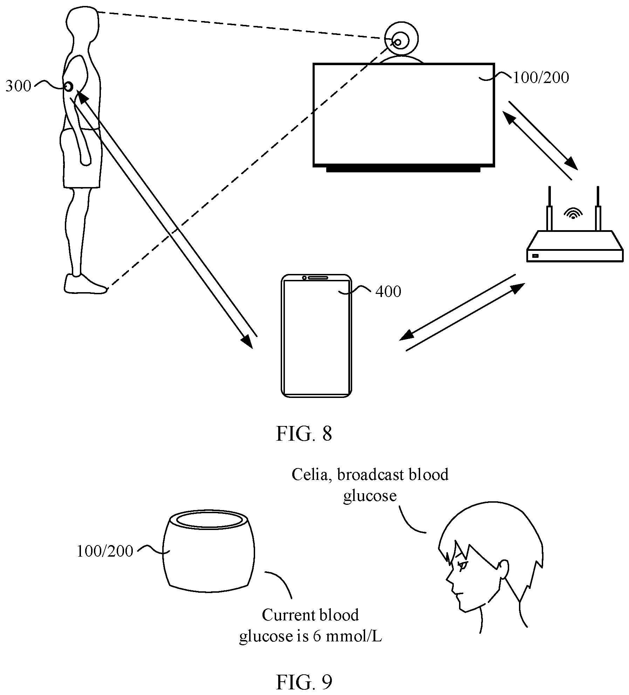

**By using an example in which the electronic device 300 is a CGM device and a body indicator monitored by the electronic device 300 is blood glucose,** **FIG. 8 to FIG. 10** **are diagrams of three application scenarios in which a user implements blood glucose broadcasting at home by using a smart home device.**

As shown in FIG. 8, the electronic device 100 and the electronic device 200 may be a same device, that is, a smart display. The electronic device 300 may be a CGM device worn by the user. The electronic device 400 may be a mobile phone of the user. In addition, a communication connection, for example, a Bluetooth connection, is established between the electronic device 400 and the electronic device 300.

In the application scenario shown in FIG. 8, the electronic device 100 may obtain an image of the user by using a camera. After recognizing a specified posture (for example, a first posture) of the user, the electronic device 100 (or the electronic device 200) may obtain and display a blood glucose value obtained by the electronic device 300. The electronic device 100 may send a blood glucose monitoring request to a home gateway, for example, a router, and the home gateway sends the blood glucose monitoring request to the electronic device 400. Then, the electronic device 400 sends the blood glucose monitoring request to the electronic device 300 through the Bluetooth connection. Then, the electronic device 300 returns an obtained blood glucose value to the electronic device 400. Then, the electronic device 400 returns the blood glucose value to the electronic device 100 by using the home gateway. The electronic device 100 displays the blood glucose value of the user or outputs alarm information when the blood glucose value exceeds a preset range.

It may be understood that, in addition to communicating with the electronic device 300 by sending data to the electronic device 300 by using the home gateway and the mobile phone, the electronic device 100 may alternatively communicate with the electronic device 300 in another manner. For example, the electronic device 100 may alternatively directly establish a communication connection to the electronic device 300, and communicate with the electronic device 300 through the communication connection. In this way, the electronic device 100 may directly send the blood glucose monitoring request to the electronic device 300, and the electronic device 300 may directly return the blood glucose value to the electronic device 100.

It can be learned that, in the application scenario shown in FIG. 8, the user may display a specified posture to a smart home device with a camera at home, to trigger the CGM device to return the obtained blood glucose value to the smart home device for displaying, thereby meeting a requirement of the user for monitoring the blood glucose of the patient.

As shown in FIG. 9, the electronic device 100 and the electronic device 200 may be a same device, that is, a smart speaker.

In the application scenario shown in FIG. 9, the user may initiate a voice instruction "Celia, broadcast blood glucose" to the electronic device 100 (or the electronic device 200), and the electronic device 200 (or the electronic device 100) may broadcast a blood glucose status of the patient "Current blood glucose is 6 mmol/L".

It may be understood that the electronic device 100 may directly communicate with the electronic device 300, send the blood glucose monitoring request to the electronic device 300, and obtain the blood glucose value obtained by the electronic device 300. Alternatively, the electronic device 100 may indirectly communicate with the electronic device 300 by using a home gateway and a mobile phone in an application scenario similar to that shown in FIG. 8.

It can be learned that, in the application scenario shown in FIG. 9, the user may directly learn the blood glucose status of the patient by performing voice interaction with the smart speaker.

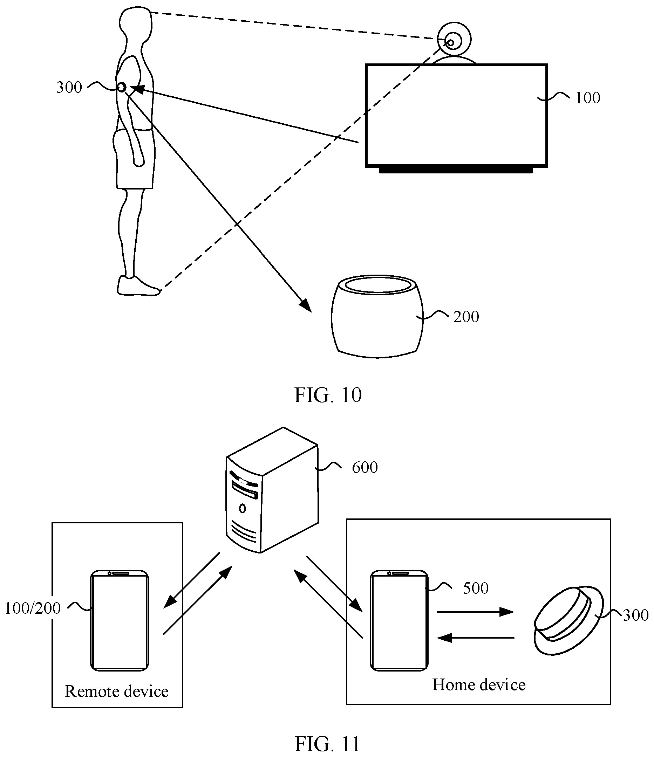

As shown in FIG. 10, the electronic device 100 may be a smart display, the electronic device 200 may be a smart speaker, and the electronic device 300 may be a CGM device.

In the application scenario shown in FIG. 10, the electronic device 100 may obtain an image of the user by using a camera, and after recognizing a specified posture of the user, control the electronic device 200 to broadcast, by using voice, the blood glucose value obtained by the electronic device 300 or indication information of the blood glucose value. The electronic device 100 may directly send the blood glucose monitoring request to the electronic device 300, and then the electronic device 300 sends the obtained blood glucose value to the electronic device 200.

It may be understood that both the electronic device 100 and the electronic device 200 may indirectly communicate with the electronic device 300 by using a home gateway and a mobile phone in an application scenario similar to that shown in FIG. 8. This is not limited in this embodiment of this application.

It can be learned that, in the application scenario shown in FIG. 10, the user may display a specified posture to the smart display, to trigger the smart speaker to broadcast, by using voice, the blood glucose value obtained by the CGM device, thereby meeting a personalized blood glucose monitoring requirement of the user.

(4) The user remotely monitors the body indicator of the patient.

For some patients requiring remote monitoring by family members, the family members of the patients may remotely initiate, by using electronic devices such as a mobile phone, a watch, a tablet, and a computer, a body indicator monitoring request to the electronic device 300 worn by the patients, to remotely obtain a body indicator status of the patients, so that body indicator monitoring is not limited by a distance, and the user can monitor the body indicator status of the patient anytime and anywhere.

**FIG. 11** **is a diagram of a scenario in which a user remotely obtains a body indicator status of a patient by using a mobile phone.**

As shown in FIG. 11, the electronic device 100 and the electronic device 200 are a same device, for example, a mobile phone of the user. The electronic device 300 is a device that is worn by the patient and that is configured to monitor a body indicator. A communication connection, for example, a Bluetooth connection, is established between the electronic device 300 and a mobile phone of the patient, that is, an electronic device 500. The electronic device 100 may communicate with the electronic device 500 by using a cloud server 600. The electronic device 100 is a remote device, and the electronic device 500 and the electronic device 300 are home devices.

In the application scenario shown in FIG. 11, the electronic device 100 may receive a user operation of monitoring a body indicator by the user, generate a body indicator monitoring request, and send the body indicator monitoring request to the electronic device 500 by using the cloud server 600. Then, the electronic device 500 sends the body indicator monitoring request to the electronic device 300. Then, the electronic device 300 returns an obtained value of the body indicator to the electronic device 500, and the electronic device 500 forwards the value to the electronic device 100 by using the cloud server 600. Then, the electronic device 100 outputs the value of the body indicator, or outputs alarm information when the value of the body indicator exceeds a preset range.

It may be understood that, if the patient further wears wearable devices such as a mobile phone, a watch, and a headset, the home devices may further include these wearable devices. If the patient is home, the home devices may further include smart home devices at home. In this case, the electronic device 300 may alternatively relay, by using another home device other than the mobile phone, data exchanged with the electronic device 100.

It can be learned that, in the application scenario shown in FIG. 11, the user may remotely obtain, by using a device carried by the user, the value that is of the body indicator and that is obtained by a device worn by the patient, to remotely monitor the body status of the patient.

**(5)** The patient implements body indicator monitoring by using a device that can be in contact with skin of the patient, such as a headset, a watch, or glasses.

Considering that the human skin has a conductive characteristic, if the electronic device 100 and/or the electronic device 200 are/is a device that can be in contact of the skin of the patient, the electronic device 300 that can be in contact with the skin of the patient and that is also worn by the patient may communicate with the electronic device 100 and/or the electronic device 200 through the human skin. The human skin is used as a communication medium, so that leakage of the value that is of the body indicator and that is obtained by the electronic device 300 can be avoided, and privacy of the patient is protected. In addition, devices can directly communicate with each other, to reduce trouble of data relay by using another device.

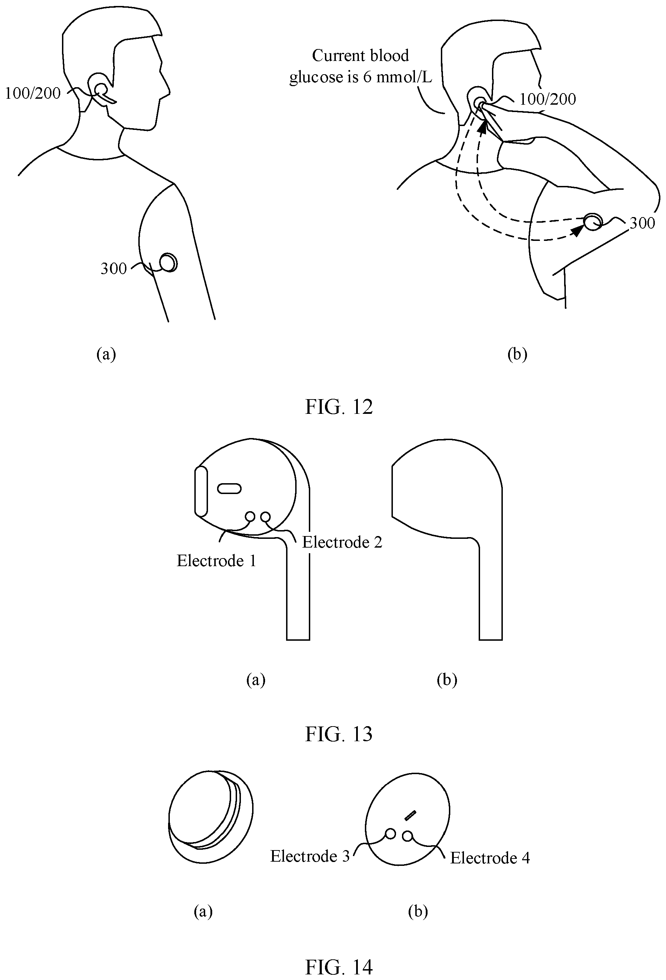

**FIG. 12** **is a diagram of a scenario in which a patient implements blood glucose broadcasting by using a headset.**

As shown in (a) in FIG. 12, the electronic device 100 and the electronic device 200 are a same device, that is, a headset worn by the patient, and the electronic device 300 is a CGM device worn by the patient.

As shown in (b) in FIG. 12, the electronic device 100 may receive a user operation, for example, a double-tap operation, generate a blood glucose monitoring request, and send the blood glucose monitoring request to the electronic device 300 through human skin. Then, the electronic device 300 returns an obtained blood glucose value to the electronic device 100 through the human skin. The electronic device 100 broadcasts the obtained blood glucose value by using voice, for example, broadcasts "Current blood glucose is 6 mmol/L".

It can be learned that, in the application scenario shown in FIG. 12, a human body may be used as a communication carrier to implement low-power, high-reliability, stable, and private data exchange. The user can learn a blood glucose status of the user only by performing a simple operation on a device, thereby facilitating an operation of the user.

**FIG. 13 and FIG. 14** **are respectively diagrams of structures of an electronic device 100 (or an electronic device 200) and an electronic device 300 in the application scenario shown in** **FIG. 12****.**

As shown in FIG. 13, (a) in FIG. 13 shows a side of the electronic device 100 facing the patient, and (b) in FIG. 13 shows a side of the electronic device 100 facing away from the patient. When the patient wears the electronic device 100, the side shown in (a) in FIG. 13 is in contact with skin of the patient. The electronic device 100 may include an electrode 1 and an electrode 2. The electrode 1 may be configured to send data to the outside, and the electrode 2 may be configured to receive data transmitted through the skin.

In other words, the electronic device 100 may send the blood glucose monitoring request to the electronic device 300 by using the electrode 1, and obtain, by using the electrode 2, the blood glucose value returned by the electronic device 300.

As shown in FIG. 14, (a) in FIG. 14 shows a side of the electronic device 300 facing away from the patient, and (b) in FIG. 14 shows a side of the electronic device 300 facing the patient. When the patient wears the electronic device 100, the side shown in (b) in FIG. 14 is in contact with skin of the patient. The electronic device 300 may include an electrode 3 and an electrode 4. The electrode 3 may be configured to send data to the outside, and the electrode 4 may be configured to receive data transmitted through the skin.

In other words, the electronic device 300 may receive, by using the electrode 4, the blood glucose monitoring request sent by the electronic device 100, and send the blood glucose value to the electronic device 100 by using the electrode 3.

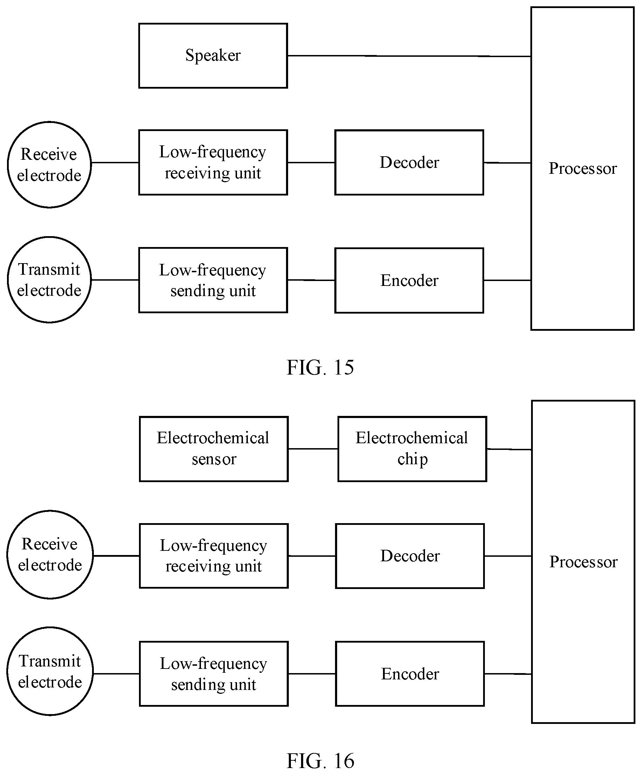

**FIG. 15** **is a diagram of a communication module of the electronic device 100 (or the electronic device 200) in the application scenario shown in** **FIG. 12****.**

As shown in FIG. 15, the electronic device 100 may include a processor, an encoder, a decoder, a low-frequency receiving unit, a low-frequency sending unit, a transmit electrode, a receive electrode, and a speaker.

After the electronic device 100 receives the user operation of the user, the processor may generate the blood glucose monitoring request, then the encoder encodes the blood glucose monitoring request with a low-frequency (10 kHz to 100 MHz) carrier, and sends obtained data to the low-frequency sending unit, and then the low-frequency sending unit sends the data by using the transmit electrode. In addition, the low-frequency receiving unit may receive, by using the receive electrode, data transmitted through the human skin, and then the decoder decodes the data to obtain a blood glucose value. Then, the processor sends, to the speaker for broadcasting, the blood glucose value or alarm information generated when the blood glucose value exceeds a preset range.

It may be understood that the receive electrode and the transmit electrode may be a same electrode, and the electronic device 100 may reuse one electrode, to send data and receive data by using the same electrode. This is not limited in this embodiment of this application. In addition, FIG. 15 shows a structure of the communication module of the electronic device 100 described by using an example in which the electronic device 100 is a headset. The communication module of the electronic device 100 may further include more or fewer modules. For example, in another embodiment of this application, if the electronic device 100 is a watch, the speaker is an optional module. The electronic device 100 may further include a display module, and display, by using the display module, a blood glucose value or alarm information generated when the blood glucose value exceeds a preset range.

**FIG. 16** **is a diagram of a communication module of the electronic device 300 in the application scenario shown in** **FIG. 12****.**

As shown in FIG. 16, the electronic device 300 may include a processor, an encoder, a decoder, a low-frequency receiving unit, a low-frequency sending unit, a transmit electrode, a receive electrode, an electrochemical chip, and an electrochemical sensor.

The electronic device 300 may obtain data of the patient by using the electrochemical sensor, and then convert the obtained data by using the electrochemical chip, to obtain a skin temperature, a blood glucose value, and the like of the patient through calculation. In addition, the electronic device 300 may obtain, by using the receive electrode, data transmitted through the human skin, and decode the data by using the decoder to obtain the blood glucose monitoring request. Then, the processor sends an obtained blood glucose value of the patient to the encoder based on the blood glucose monitoring request. The encoder encodes the blood glucose value with a low-frequency carrier, and sends obtained data to the low-frequency sending unit. Then, the low-frequency sending unit sends the data by using the transmit electrode.

It may be understood that, similar to the description in FIG. 15, the receive electrode and the transmit electrode may be a same electrode, and the electronic device 300 may reuse one electrode, to send data and receive data by using the same electrode. This is not limited in this embodiment of this application.

It should be noted that, in the application scenario (5), the electronic device 100 and the electronic device 200 may alternatively be different devices. The electronic device 100 may communicate with the electronic device 300 through the skin, and the electronic device 200 may also communicate with the electronic device 300 through the skin. For example, the electronic device 100 may be a headset, and the electronic device 300 may be a watch. The user may view, on the watch by initiating a user operation of blood glucose broadcasting to the headset, a blood glucose value obtained by the CGM device, or alarm information output when the blood glucose is abnormal.

It may be understood that, the skin communication manner shown in FIG. 12 may be used not only for blood glucose monitoring, and other devices that can monitor a body indicator and that are worn by the patient may communicate, in the skin communication manner, with other devices that can be in contact with the skin of the patient. A principle thereof is similar to that of FIG. 12 to FIG. 16, and details are not described herein again.

It should be noted that, in this embodiment of this application, manners of communication among the electronic device 100, the electronic device 200, and the electronic device 300 may include both a human body communication manner and another communication manner. For example, the electronic device 100 may communicate with the electronic device 300 by using human skin, to transmit a body indicator monitoring request, and the electronic device 300 may transmit a value of a body indicator to the electronic device 200 in a communication manner such as Bluetooth or Wi-Fi.

It may be understood that the implementations in the foregoing plurality of application scenarios may be combined with each other. For example, the implementation of triggering, by using different body movements, body indicator broadcasting in different modes in the application scenario 1 may be used in combination with the implementation of human skin communication in the application scenario 5. In other words, the electronic device 100 and/or the electronic device 200 may communicate with the electronic device 300 by using human skin, and different body movements performed by the user on the electronic device 100 may trigger the electronic device 200 to output the value of the body indicator or output the alarm information.

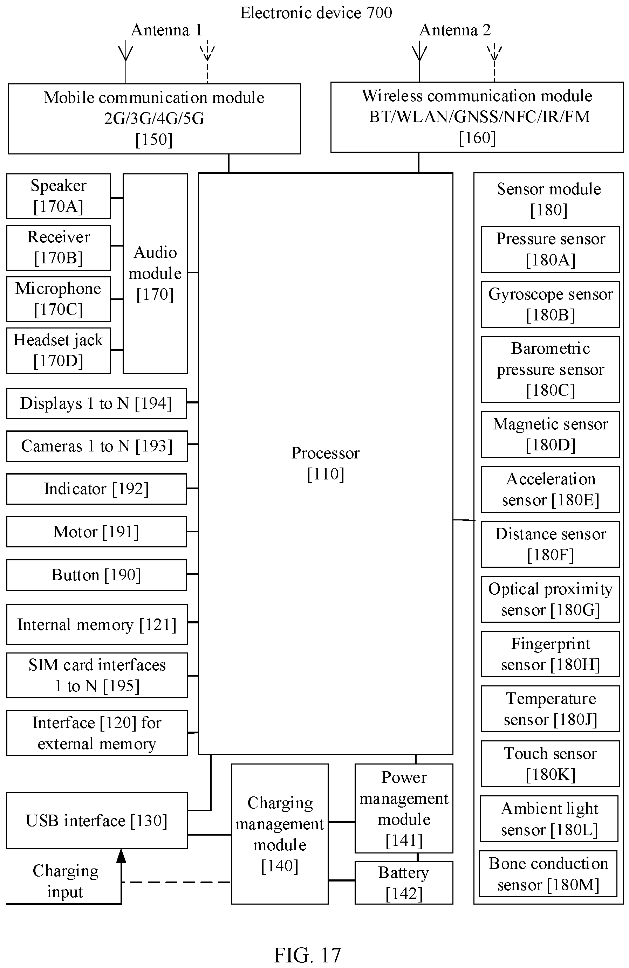

**FIG. 17** **is a diagram of a hardware structure of an electronic device 700.**

The electronic device 700 may be a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device, an in-vehicle device, a smart home device, and/or a smart city device, and a specific type of the electronic device is not limited in this embodiment of this application.

The electronic device 700 may include a processor 110, an interface 120 for external memory, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 700. In some other embodiments of this application, the electronic device 700 may include more or fewer components than those shown in the figure, or some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a timing signal, to complete control of instruction fetching and instruction execution.

A memory may be further disposed in the processor 110, and is configured to store instructions and data. In some embodiments, the memory in the processor 110 is a cache memory. The memory may store an instruction or data that has been used or cyclically used by the processor 110. If the processor 110 needs to use the instruction or the data again, the processor may directly invoke the instruction or the data from the memory. This avoids repeated access, reduces waiting time of the processor 110, and therefore improves system efficiency.

The charging management module 140 is configured to receive a charging input from a charger. The charger may be a wireless charger or a wired charger. In some embodiments of wired charging, the charging management module 140 may receive a charging input of a wired charger through the USB interface 130. In some embodiments of wireless charging, the charging management module 140 may receive a wireless charging input through a wireless charging coil of the electronic device 700. When the charging management module 140 charges the battery 142, the power management module 141 may further supply power to the electronic device.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same component.

A wireless communication function of the electronic device 700 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 700 may be configured to cover one or more communication bands. Different antennas may be further reused, to improve antenna utilization. For example, the antenna 1 may be reused as a diversity antenna of a wireless local area network. In some other embodiments, the antenna may be used in combination with a tuning switch.

The mobile communication module 150 may provide a wireless communication solution that is applied to the electronic device 700 and that includes 2G/3G/4G/5G or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules in the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 170A, the receiver 170B, or the like), or displays an image or a video through the display 194. In some embodiments, the modem processor may be an independent component. In some other embodiments, the modem processor may be independent of the processor 110, and is disposed in a same device as the mobile communication module 150 or another functional module.

The wireless communication module 160 may provide a wireless communication solution that is applied to the electronic device 700 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, human skin communication, and the like. The wireless communication module 160 may be one or more components integrating at least one communication processing module. The wireless communication module 160 receives an electromagnetic wave through the antenna 2, performs demodulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the electronic device 700, the antenna 1 is coupled to the mobile communication module 150, and the antenna 2 is coupled to the wireless communication module 160, so that the electronic device 700 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or satellite based augmentation systems (satellite based augmentation systems, SBAS).

The electronic device 700 implements a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs that execute program instructions to generate or change display information.

The display 194 is configured to display an image, a video, and the like. The display 194 includes a display panel. The display panel may use a liquid crystal display (liquid crystal display, LCD). The display panel may alternatively be manufactured by using an organic light-emitting diode (organic light-emitting diode, OLED), an active-matrix organic light emitting diode (active-matrix organic light emitting diode, AMOLED), a flex light-emitting diode (flex light-emitting diode, FLED), a mini-LED, a micro-LED, a micro-OLED, a quantum dot light emitting diode (quantum dot light emitting diode, QLED), or the like. In some embodiments, the electronic device may include one or N displays 194. N is a positive integer greater than 1.

The electronic device 700 may implement a shooting function through the ISP, the camera 193, the video codec, the GPU, the display 194, the application processor, and the like.

The ISP is configured to process data fed back by the camera 193. For example, during photographing, a shutter is opened, and light is transmitted to a photosensitive element of the camera through a lens. An optical signal is converted into an electrical signal. The photosensitive element of the camera transmits the electrical signal to the ISP for processing, to convert the electrical signal into an image visible to naked eyes. The ISP may further perform algorithm optimization on noise and brightness of the image. The ISP may further optimize parameters such as exposure and a color temperature of a photographing scene. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format, for example, RGB or YUV. In some embodiments, the electronic device 700 may include one or N cameras 193, where N is a positive integer greater than 1.

The digital signal processor is configured to process a digital signal, and may process another digital signal in addition to the digital image signal. For example, when the electronic device 700 selects a frequency, the digital signal processor is configured to perform Fourier transformation and the like on frequency energy.

The video codec is configured to compress or decompress a digital video. The electronic device 700 may support one or more video codecs. In this way, the electronic device 700 may play or record videos in a plurality of coding formats, for example, moving picture experts group (moving picture experts group, MPEG) 1, MPEG 2, MPEG 3, and MPEG 4.

The NPU is a neural-network (neural-network, NN) computing processor, and quickly processes input information with reference to a structure of a biological neural network, for example, a transfer mode between human brain neurons, and may further continuously perform self-learning. Applications such as intelligent cognition of the electronic device 700, for example, image recognition, facial recognition, voice recognition, and text understanding, may be implemented through the NPU.

The internal memory 121 may include one or more random access memories (random access memory, RAM), and one or more non-volatile memories (non-volatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a 5th generation DDR SDRAM generally referred to as a DDR5 SDRAM), or the like. The non-volatile memory may include a magnetic disk storage device and a flash memory (flash memory).

According to an operating principle, the flash memory may be classified into a NOR FLASH, a NAND FLASH, a 3D NAND FLASH, or the like; according to a quantity of potential levels per cell, the flash memory may be classified into a single-level cell (single-level cell, SLC), a multi-level cell (multi-level cell, MLC), a triple-level cell (triple-level cell, TLC), a quad-level cell (quad-level cell, QLC), or the like; and according to storage specifications, the flash memory may be classified into a universal flash storage (English: universal flash storage, UFS), an embedded multimedia memory card (embedded multimedia card, eMMC), or the like.

The random access memory may be directly read and written by the processor 110, may be configured to store executable programs (for example, machine instructions) of an operating system or another running program, and may also be configured to store data of a user and an application, and the like.

The non-volatile memory may also store executable programs, data of the user and the application, and the like, and may be loaded into the random access memory in advance, to be directly read and written by the processor 110.

The interface 120 for external memory may be configured to connect to an external non-volatile memory, to expand a storage capability of the electronic device 700. The external non-volatile memory communicates with the processor 110 through the interface 120 for external memory, to implement a data storage function. For example, files such as music and videos are stored in the external non-volatile memory.

The electronic device 700 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be further configured to encode and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 700 may listen to music or answer a hands-free call by using the speaker 170A.

The receiver 170B, also referred to as an "earpiece", is configured to convert an electrical audio signal into a sound signal. When a call is answered or voice information is received through the electronic device 700, the receiver 170B may be put close to a human ear to listen to voice.

The microphone 170C, also referred to as a "mike" or a "mic", is configured to convert a sound signal into an electrical signal. When making a call or sending a voice message, a user may make a sound near the microphone 170C through the mouth of the user, to input a sound signal to the microphone 170C. At least one microphone 170C may be disposed in the electronic device 700. In some other embodiments, two microphones 170C may be disposed in the electronic device 700, to obtain a sound signal and further implement a noise reduction function. In some other embodiments, three, four, or more microphones 170C may alternatively be disposed in the electronic device 700, to obtain a sound signal, reduce noise, identify a sound source, implement a directional recording function, and the like.

The headset jack 170D is configured to connect to a wired headset. The headset jack 170D may be the USB interface 130, or may be a 3.5 mm open mobile electronic device platform (open mobile terminal platform, OMTP) standard interface or a cellular telecommunications industry association of the USA (cellular telecommunications industry association of the USA, CTIA) standard interface.

The gyroscope sensor 180B may be configured to determine a motion posture of the electronic device 700. In some embodiments, an angular velocity of the electronic device 700 around three axes (that is, axes x, y, and z) may be determined by using the gyroscope sensor 180B. The gyroscope sensor 180B may be configured to implement image stabilization during shooting. For example, when the shutter is pressed, the gyroscope sensor 180B detects an angle at which the electronic device 700 jitters, calculates, based on the angle, a distance for which a lens module needs to compensate, and allows the lens to cancel the jitter of the electronic device 700 through reverse motion, to implement image stabilization. The gyroscope sensor 180B may also be configured to: navigate and sense a game scene.

The acceleration sensor 180E may detect magnitudes of accelerations of the electronic device 700 in various directions (generally three axes). When the electronic device 700 is still, a magnitude and a direction of gravity may be detected. The acceleration sensor 180E may be further configured to recognize a posture of the electronic device, and is used in an application such as switching between a landscape mode and a portrait mode or a pedometer.

The touch sensor 180K is also referred to as a "touch component". The touch sensor 180K may be disposed on the display 194, and the touch sensor 180K and the display 194 constitute a touchscreen, which is also referred to as a "touch screen". The touch sensor 180K is configured to detect a touch operation performed on or near the touch sensor. The touch sensor may transfer the detected touch operation to the application processor to determine a type of the touch event. A visual output related to the touch operation may be provided through the display 194. In some other embodiments, the touch sensor 180K may also be disposed on a surface of the electronic device 700 at a position different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also come into contact with a human pulse to receive a blood pressure beating signal. In some embodiments, the bone conduction sensor 180M may alternatively be disposed in a headset, to combine into a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or a touch-type button. The electronic device 700 may receive a key input, and generate a key signal input related to user settings and function control of the electronic device 700.

The motor 191 may generate a vibration prompt. The motor 191 may be configured to provide an incoming call vibration prompt, or may be configured to provide a touch vibration feedback. For example, touch operations performed on different applications (for example, photographing and audio playing) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. A touch vibration feedback effect may be further customized.

The indicator 192 may be an indicator light, and may indicate a charging status and a power level change, or may indicate a message, a missed call, a notification, and the like.

In this embodiment of this application, the electronic device 700 may be the electronic device 100 or the electronic device 200 mentioned above.

**In some implementations, when the electronic device 700 is the electronic device 100,**
the processor 110 may be configured to generate a body indicator monitoring request based on a user operation.

The user operation may be an operation performed by a user on the button 190, or the user operation may be a touch operation performed by the user on the display 194. The electronic device 700 may detect the touch operation by using the touch sensor 180K. Alternatively, the user operation may be a body movement of the user. The electronic device 700 may detect a body movement of the user by using the gyroscope sensor 180B or the acceleration sensor 180E. Alternatively, the user operation may be a posture of the user. The electronic device 700 may obtain a posture of the user by using the camera 193.

The electronic device 700 may send the body indicator monitoring request by using the mobile communication module 150 or the wireless communication module 160. In addition, if the electronic device 700 performs communication by using human skin, the electronic device 700 may send the body indicator monitoring request by using an electrode (not shown in the figure).

**In some implementations, when the electronic device 700 is the electronic device 200,**
the electronic device 700 may obtain, by using the mobile communication module 150 or the wireless communication module 160, a value that is of a body indicator and that is obtained by the electronic device 300. In addition, if the electronic device 700 performs communication by using human skin, the electronic device 700 may receive the value of the body indicator by using an electrode (not shown in the figure).

When the electronic device 700 outputs the value of the body indicator or alarm information, the electronic device 700 may display the value of the body indicator or the alarm information by using the display 194, broadcast the value of the body indicator or the alarm information by using the speaker 170A, and broadcast the value of the body indicator or the alarm information by outputting vibration by using the motor 191.

The electronic device may be a portable terminal device carrying Harmony, iOS, Android, Microsoft, or another operating system, for example, a mobile phone, a tablet computer, or a wearable device; or may be a non-portable terminal device, for example, a laptop (Laptop) computer having a touch-sensitive surface or a touch panel, or a desktop computer having a touch-sensitive surface or a touch panel. A software system of the electronic device 700 may use a layered architecture, an event-driven architecture, a microkernel architecture, a microservice architecture, or a cloud architecture. In an embodiment of the present invention, a software structure of the electronic device 700 is described by using an Android system with a layered architecture as an example.

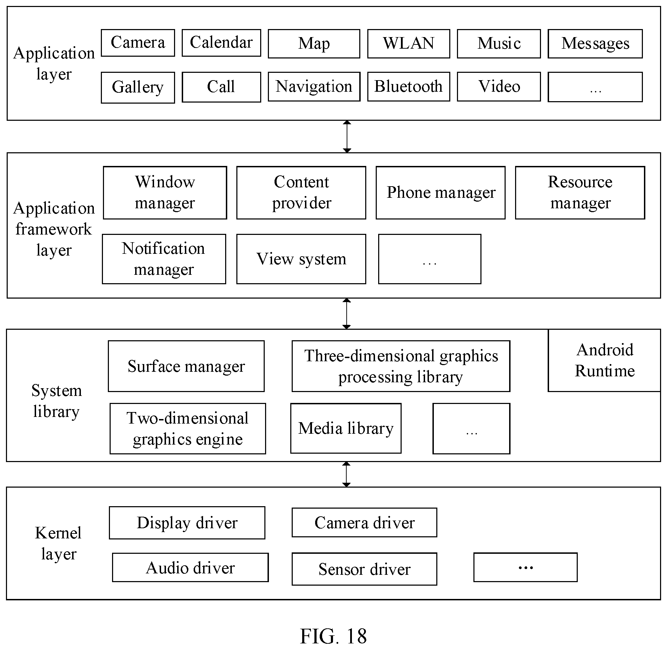

**FIG. 18** **is a block diagram of a software structure of an electronic device 700 according to an embodiment of this application.**

In the layered architecture, software is divided into several layers, and each layer has a clear role and task. The layers communicate with each other through a software interface. In some embodiments, the Android system is divided into four layers: an application layer, an application framework layer, an Android Runtime (Android Runtime) and a system library, and a kernel layer from top to bottom.

The application layer may include a series of application packages.

As shown in FIG. 18, the application packages may include applications such as Camera, Gallery, Calendar, Call, Map, Navigation, WLAN, Bluetooth, Music, Video, and Messages.

The application framework layer provides an application programming interface (application programming interface, API) and a programming framework for an application at the application layer. The application framework layer includes some predefined functions.

As shown in FIG. 18, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to: store and obtain data, and enable the data to be accessible to an application. The data may include a video, an image, audio, calls made and answered, a browse history and a bookmark, a personal address book, and the like.

The view system includes visual controls such as a text display control and a picture display control. The view system may be configured to construct an application. A display interface may include one or more views. For example, a display interface including a message notification icon may include a text display view and a picture display view.

The phone manager is configured to provide a communication function for the electronic device 700, for example, management of a call status (including answering, declining, or the like).

The resource manager provides various resources such as a localized character string, an icon, a picture, a layout file, and a video file for an application.

The notification manager enables an application to display, in the status bar, notification information, which may be used for conveying a notification-type message that may automatically disappear after a short stay without user interaction. For example, the notification manager is configured to: notify download completion, give a message notification, and the like. The notification manager may alternatively be a notification that appears in a top status bar of the system in a form of a chart or scroll bar text, for example, a notification of an application running on the background or a notification that appears on the screen in a form of a dialog window. For example, text information is prompted in the status bar, an alert tone is made, the electronic device vibrates, or an indicator blinks.

The Android Runtime includes a core library and a virtual machine. The Android Runtime is responsible for scheduling and management of the Android system.

The core library includes two parts: a function that needs to be called in Java language and a core library of Android.

The application layer and the application framework layer run in the virtual machine. The virtual machine executes Java files of the application layer and the application framework layer as binary files. The virtual machine is configured to execute functions such as object life cycle management, stack management, thread management, security and exception management, and garbage collection.

The system library may include a plurality of functional modules, for example, a surface manager (surface manager), media libraries (Media Libraries), a three-dimensional graphics processing library (for example, an OpenGL ES), a 2D graphics engine (for example, an SGL), and the like.

The surface manager is configured to: manage a display subsystem and provide fusion of 2D and 3D layers for a plurality of applications.

The media library supports playback and recording in a plurality of commonly used audio and video formats, and static image files. The media library may support a plurality of audio and video coding formats, for example, MPEG 4, H.264, MP3, AAC, AMR, JPG, and PNG.

The three-dimensional graphics processing library is configured to implement three-dimensional graphics drawing, image rendering and compositing, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes working procedures of software and hardware of the electronic device 700 by using examples with reference to a photo capture scenario.

When the touch sensor 180K receives a touch operation, a corresponding hardware interruption is sent to the kernel layer. The kernel layer processes the touch operation into an original input event (including information such as coordinates of touch and a timestamp of the touch operation). The original input event is stored at the kernel layer. The application framework layer obtains the original input event from the kernel layer, and identifies a control corresponding to the input event. Using an example in which the touch operation is a touch tapping operation, and a control corresponding to the tapping operation is a control of an icon of the camera application, the camera application invokes an interface of the application framework layer, and the camera application is started, so that a camera driver is started by invoking the kernel layer, and a static image or a video is captured by using the camera 193.

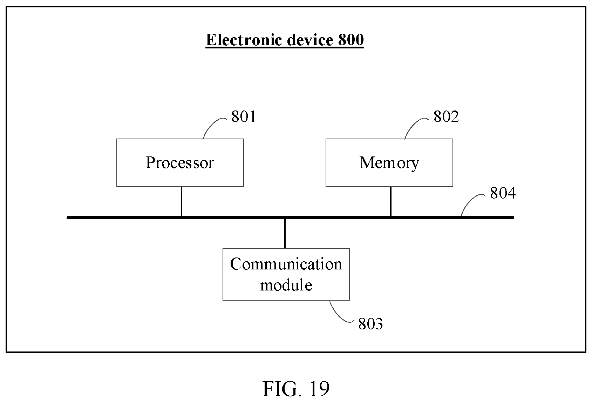

**FIG. 19** **is a diagram of a hardware structure of an electronic device 800 according to an embodiment of this application.**

As shown in FIG. 19, the electronic device 800 may include components such as a processor 801, a memory 802, and a communication module 803. These components may be connected through a bus 804 or in another manner. In FIG. 19, connection through a bus is used as an example. The bus 804 is configured to implement connection and communication among the processor 801, the memory 802, and the communication module 803.

The processor 801 may include one or more processing units. The processor 801 may be configured to: provide a computing and control capability, and support running of the entire electronic device 800.

The memory 802 may be configured to store various software programs and/or a plurality of groups of instructions. Specifically, the memory 802 may include a high-speed random access memory, and may further include a non-volatile memory, for example, one or more disk storage devices, a flash memory device, or another non-volatile solid-state storage device.

The communication module 803 may be configured to communicate with another communication device by the electronic device 800. Specifically, the communication module 803 may include a communication interface. The communication interface may be a 3G communication interface, a long term evolution (LTE) (4G) communication interface, a 5G communication interface, a WLAN communication interface, a WAN communication interface, a human skin communication interface, or the like. The communication interface is not limited to a wireless communication interface. The electronic device 800 may be also configured with a wired communication interface to support wired communication.

In this embodiment of this application, the electronic device 800 may be the electronic device 100 or the electronic device 200. The processor 801 of the electronic device 800 may be configured to perform steps performed by the electronic device 100 or the electronic device 200 in the embodiments. The memory 802 may be configured to store software or program code required for all or some functions of the electronic device 100 or the electronic device 200 in the foregoing method embodiments. The communication module 803 may be configured to implement a process of communication between the electronic device 100 or the electronic device 200 and another device in the foregoing method embodiments.

It should be noted that the electronic device 800 shown in FIG. 19 is merely an implementation of this embodiment of this application. In actual application, the electronic device 800 may include more or fewer components than those shown in the figure, or combine some components, or have different component arrangements. This is not limited herein.

It should be understood that the steps in the foregoing method embodiments may be completed by using an integrated logic circuit of hardware in the processor or instructions in a form of software. The steps of the method disclosed with reference to embodiments of this application may be directly performed by a hardware processor, or may be performed by a combination of hardware and software modules in a processor.

This application further provides an electronic device, and the electronic device may include a memory and a processor. The memory may be configured to store a computer program. The processor may be configured to invoke the computer program in the memory, so that the electronic device performs the method performed by the electronic device 100 or the electronic device 200 in any one of the foregoing embodiments.

This application further provides a chip system. The chip system includes at least one processor, configured to implement functions in the method performed by the electronic device 100 or the electronic device 200 in any one of the foregoing embodiments.

In a possible design, the chip system further includes a memory. The memory is configured to store program instructions and data. The memory is located inside the processor or outside the processor.

The chip system may include a chip, or may include a chip and another discrete component.

Optionally, there may be one or more processors in the chip system. The processor may be implemented by using hardware, or may be implemented by using software. When the processor is implemented by using the hardware, the processor may be a logic circuit, an integrated circuit, or the like. When implemented by using software, the processor may be a general-purpose processor, and is implemented by reading software code stored in the memory.

Optionally, there may also be one or more memories in the chip system. The memory may be integrated with the processor, or may be separated from the processor. This is not limited in this embodiment of this application. For example, the memory may be a non-transitory processor such as a read-only memory ROM, and the memory and the processor may be integrated on a same chip, or may be disposed on different chips respectively. A type of the memory and an arrangement manner of the memory and the processor are not limited in this embodiment of this application.

For example, the chip system may be a field programmable gate array (field programmable gate array, FPGA), an application-specific integrated circuit (application-specific integrated circuit, ASIC), a system on chip (system on chip, SoC), a central processing unit (central processor unit, CPU), a network processor (network processor, NP), a digital signal processor (digital signal processor, DSP), a microcontroller unit (microcontroller unit, MCU), a programmable logic device (programmable logic device, PLD), or another integrated chip.

This application further provides a computer program product. The computer program product includes a computer program (which may also be referred to as code or instructions). When the computer program is run, a computer is enabled to perform the method performed by the electronic device 100 or the electronic device 200 in any one of the foregoing embodiments.

This application further provides a computer-readable storage medium. The computer-readable storage medium stores a computer program (which may also be referred to as code or instructions). When the computer program is run, a computer is enabled to perform the method performed by the electronic device 100 or the electronic device 200 in any one of the foregoing embodiments.

It should be understood that, the processor in this embodiment of this application may be an integrated circuit chip, and has a signal processing capability. In an implementation process, the steps in the foregoing method embodiments may be completed by using an integrated logic circuit of hardware in the processor or instructions in a form of software. The processor may be a general-purpose processor, a digital signal processor (digital signal processor, DSP), an application-specific integrated circuit (application-specific integrated circuit, ASIC), a field programmable gate array (field programmable gate array, FPGA) or another programmable logic device, a discrete gate or a transistor logic device, or a discrete hardware component. The methods, steps, and logic block diagrams disclosed in embodiments of this application may be implemented or executed. The general-purpose processor may be a microprocessor, or the processor may be any conventional processor or the like. The steps of the methods disclosed with reference to embodiments of this application may be directly performed by a hardware decoding processor, or may be performed by using a combination of hardware and software modules in the decoding processor. The software module may be located in a mature storage medium in the art, such as a random access memory, a flash memory, a read-only memory, a programmable read-only memory, an electrically erasable programmable memory, or a register. The storage medium is located in the memory, and the processor reads information in the memory and completes the steps in the foregoing method in combination with hardware of the processor.

In addition, an embodiment of this application further provides an apparatus. The apparatus may be specifically a component or a module, and the apparatus may include one or more processors and memories that are connected to each other. The memory is configured to store a computer program. When the computer program is executed by one or more processors, the apparatus is enabled to perform the methods in the foregoing method embodiments.

The apparatus, the computer-readable storage medium, the computer program product, or the chip provided in embodiments of this application is configured to perform the corresponding methods provided above. Therefore, for beneficial effects that can be achieved, refer to beneficial effects in the corresponding methods provided above, and details are not described herein again.

The implementations of this application may be randomly combined to achieve different technical effects.

All or some of the foregoing embodiments may be implemented by using software, hardware, firmware, or any combination thereof. When software is used to implement the embodiments, all or some of the embodiments may be implemented in a form of a computer program product. The computer program product includes one or more computer instructions. When the computer program instructions are loaded and executed on a computer, the procedures or functions according to this application are all or partially generated. The computer may be a general-purpose computer, a dedicated computer, a computer network, or other programmable apparatuses. The computer instructions may be stored in a computer-readable storage medium, or transmitted from one computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, wireless, or microwaves) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

Persons of ordinary skill in the art may understand that all or some of the procedures of the methods in the foregoing embodiments may be implemented by a computer program instructing related hardware. The program may be stored in the computer-readable storage medium. When the program is executed, the procedures in the foregoing method embodiments may be included. The foregoing storage medium includes any medium that can store program code, such as a ROM, a random access memory RAM, a magnetic disk, or an optical disc.

In conclusion, the foregoing is merely embodiments of the technical solution of the present invention, but is not intended to limit the protection scope of the present invention. Any modification, equivalent replacement, or improvement made according to the disclosure of the present invention shall fall within the protection scope of the present invention.

## Claims

1. A body indicator monitoring method, wherein the method comprises:
receiving, by a first device, a first operation;
sending, by the first device, first information to a third device in response to the first operation;
sending, by the third device, an obtained value of a first body indicator to a second device after receiving the first information; and
outputting, by the second device, the value of the first body indicator, or outputting alarm information when the value of the first body indicator exceeds a first range.

2. The method according to claim 1, wherein
when the first operation is a first body movement, the second device outputs the value of the first body indicator;
or
when the first operation is a second body movement, the second device outputs the alarm information when the value of the first body indicator exceeds the first range.

3. The method according to claim 1 or 2, wherein the first device and the third device are devices used by a same user, the first operation is a body movement, and before sending, by the third device, the obtained value of the first body indicator to the second device, the method further comprises:
receiving, by the third device, the first operation.

4. The method according to any one of claims 1 to 3, wherein before outputting, by the second device, the alarm information when the value of the first body indicator exceeds the first range, the method further comprises:
receiving, by the first device or the second device, that a value of a second body indicator of the user wearing the third device exceeds a second range, wherein the second body indicator is related to the first body indicator.

5. The method according to any one of claims 1 to 4, wherein outputting, by the second device, the value of the first body indicator specifically comprises:
displaying, by the second device, the value of the first body indicator, and/or displaying a first body indicator curve, wherein the first body indicator curve comprises a plurality of values of the first body indicator that are obtained by the third device.

6. A body indicator monitoring method, wherein a first device and a third device are devices used by a same user, and the method comprises:
sending, by the first device, first information to the third device when a value that is of a second body indicator and that is obtained by the first device exceeds a second range or the first device receives a first movement;
sending, by the third device, an obtained value of a first body indicator to a second device after receiving the first information, wherein the first body indicator is related to the second body indicator; and
outputting, by the second device, alarm information when the value of the first body indicator exceeds a first range.

7. The method according to any one of claims 1 to 6, wherein
the second device outputs the alarm information when the value of the first body indicator exceeds the first range, and
the second device is a device that is being used by the user, and the second device is a device that establishes a communication connection to the first device and/or the first device.

8. The method according to any one of claims 1 to 7, wherein the first device comprises a first electrode, the third device comprises a second electrode, and both the first electrode and the second electrode are in contact with skin of the user; and
sending, by the first device, the first information to the third device specifically comprises:
sending, by the first device, the first information to the second electrode of the third device by using the first electrode.

9. The method according to any one of claims 1 to 8, wherein the second device comprises a third electrode, the third device comprises a fourth electrode, and both the third electrode and the fourth electrode are in contact with the skin of the user; and
sending, by the third device, the obtained value of the first body indicator to the second device specifically comprises:
sending, by the third device to the third electrode of the second device by using the fourth electrode, the value that is of the first body indicator and that is obtained by the third device.

10. The method according to any one of claims 1 to 9, wherein the value of the first body indicator comprises one or more values, and the value indicates a current actual status of the first body indicator of the user, or indicates a current-historical change status of the first body indicator of the user.

11. The method according to any one of claims 1 to 10, wherein
the third device is a continuous glucose monitoring CGM device, and the first body indicator is blood glucose; or
the third device is a continuous ketone monitoring CKM device, and the first body indicator is blood ketone; or
the third device is a continuous lactate monitoring CLM device, and the first body indicator is lactate; or
the third device is an electrocardiogram patch, and the first body indicator is an electrocardiogram signal.

12. A body indicator monitoring method, wherein the method comprises:
receiving, by a first device, a first operation;
sending, by the first device, first information to a third device in response to the first operation;
receiving, by the first device, a value that is of a first body indicator and that is obtained by the third device; and
outputting, by the first device, the value of the first body indicator, or outputting alarm information when the value of the first body indicator exceeds a first range.

13. The method according to claim 12, wherein
when the first operation is a first body movement, the first device outputs the value of the first body indicator;
or
when the first operation is a second body movement, the first device outputs the alarm information when the value of the first body indicator exceeds the first range.

14. The method according to claim 12 or 13, wherein before outputting, by the first device, the alarm information when the value of the first body indicator exceeds the first range, the method further comprises:
receiving, by the first device, that a value of a second body indicator of a user wearing the third device exceeds a second range, wherein the second body indicator is related to the second body indicator.

15. The method according to any one of claims 12 to 14, wherein outputting, by the first device, the value of the first body indicator specifically comprises:
displaying, by the first device, the value of the first body indicator, and/or displaying a first body indicator curve, wherein the first body indicator curve comprises a plurality of values of the first body indicator that are obtained by the third device.

16. A body indicator monitoring method, wherein a first device and a third device are devices used by a same user, and the method comprises:
sending, by the first device, first information to the third device when a value that is of a second body indicator and that is obtained by the first device exceeds a second range or the first device receives a first movement, wherein the first information indicates the third device to send a value that is of a first body indicator and that is obtained by the third device;
receiving, by the first device, the value of the first body indicator, wherein the first body indicator is related to the second body indicator; and
outputting, by the first device, alarm information when the value of the first body indicator exceeds a first range.

17. The method according to any one of claims 12 to 16, wherein the first device comprises a first electrode, and the first electrode is in contact with skin of the user; and
sending, by the first device, the first information to the third device specifically comprises:
sending, by the first device, the first information to the third device by using the first electrode.

18. The method according to any one of claims 12 to 17, wherein the first device comprises a third electrode, and the third electrode is in contact with the skin of the user; and
receiving, by the first device, the value that is of the first body indicator and that is obtained by the third device specifically comprises:
receiving, by the first device by using the third electrode, the value that is of the first body indicator and that is obtained by the third device.

19. The method according to any one of claims 12 to 18, wherein the value of the first body indicator comprises one or more values, and the value indicates a current actual status of the first body indicator of the user, or indicates a current-historical change status of the first body indicator of the user.

20. The method according to any one of claims 12 to 19, wherein
the third device is a continuous glucose monitoring CGM device, and the first body indicator is blood glucose; or
the third device is a continuous ketone monitoring CKM device, and the first body indicator is blood ketone; or
the third device is a continuous lactate monitoring CLM device, and the first body indicator is lactate; or
the third device is an electrocardiogram patch, and the first body indicator is an electrocardiogram signal.

21. A communication system, wherein the communication system comprises a first device, a second device, and a third device, wherein
the first device is configured to: receive a first operation, and send first information to the third device in response to the first operation;
the third device is configured to send an obtained value of a first body indicator to the second device after receiving the first information; and
the second device is configured to: output the value of the first body indicator, or output alarm information when the value of the first body indicator exceeds a first range.

22. A communication system, wherein the communication system comprises a first device, a second device, and a third device, and the first device and the third device are devices used by a same user, wherein
the first device is configured to send first information to the third device when a value that is of a second body indicator and that is obtained by the first device exceeds a second range or the first device receives a first movement;
the third device is configured to send an obtained value of a first body indicator to the second device after receiving the first information, wherein the first body indicator is related to the second body indicator; and
the second device is configured to output alarm information when the value of the first body indicator exceeds a first range.

23. An electronic device, comprising a memory, one or more processors, and one or more programs, wherein when the one or more processors execute the one or more programs, the electronic device is enabled to implement the method according to any one of claims 12 to 20.

24. A computer-readable storage medium, comprising instructions, wherein when the instructions are run on an electronic device, the electronic device is enabled to perform the method according to any one of claims 12 to 20.
